(19) **Europäisches Patentamt / European Patent Office / Office européen des brevets**

(11) **EP 2 997 396 B1**

(12) # FASCICULE DE BREVET EUROPEEN

(45) Date de publication et mention de la délivrance du brevet:
**26.12.2018  Bulletin 2018/52**

(51) Int Cl.:
**G01T 1/14** *(2006.01)*    **G01N 33/483** *(2006.01)*

(21) Numéro de dépôt: **14729962.2**

(22) Date de dépôt: **15.05.2014**

(86) Numéro de dépôt international:
**PCT/FR2014/051136**

(87) Numéro de publication internationale:
**WO 2014/184498 (20.11.2014 Gazette 2014/47)**

(54) **PROCÉDÉ D'ÉVALUATION PAR BIODOSIMÉTRIE DE LA DOSE D'IRRADIATION REÇUE PAR UN INDIVIDU AYANT ÉTÉ SOUMIS A UN RAYONNEMENT IONISANT**

VERFAHREN ZUR BEURTEILUNG, MITTELS BIODOSIMETRIE, DER VON EINER PERSON UNTER DEM EINFLUSS VON IONISIERENDER STRAHLUNG AUFGENOMMENEN STRAHLENDOSIS

METHOD FOR EVALUATING, VIA BIODOSIMETRY, THE IRRADIATION DOSE RECEIVED BY A PERSON SUBJECTED TO IONIZING RADIATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.05.2013  FR 1354347**

(43) Date de publication de la demande:
**23.03.2016  Bulletin 2016/12**

(73) Titulaire: **Acubens**
**92160 Antony (FR)**

(72) Inventeurs:
- **JUKIC, Ivana**
  **75014 Paris (FR)**
- **SCHELLENBERG, Amélie**
  **75014 Paris (FR)**
- **DELIC, Jozo**
  **92340 Bourg-La-Reine (FR)**
- **TUPINIER, Arnaud**
  **94500 Champigny Sur Marne (FR)**
- **PAGET, Vincent**
  **91380 Chilly Mazarin (FR)**
- **FEDOR, Yohann**
  **92260 Fontenay-aux-Roses (FR)**
- **MORAT, Luc**
  **92350 Le Plessis Robinson (FR)**
- **UGOLIN, Nicolas**
  **75002 Paris (FR)**
- **FLACK, Caroline**
  **92220 Bagneux (FR)**

- **CHEVILLARD, Sylvie**
  **75005 Paris (FR)**
- **LEFEVRE, Emilie**
  **91540 Mennecy (FR)**

(74) Mandataire: **Gevers & Orès**
**41 avenue de Friedland**
**75008 Paris (FR)**

(56) Documents cités:
**US-B1- 7 625 708**

- **CHRISTOPHE E. REDON ET AL: "The Use of Gamma-H2AX as a Biodosimeter for Total-Body Radiation Exposure in Non-Human Primates", PLOS ONE, vol. 5, no. 11, 23 novembre 2010 (2010-11-23), page e15544, XP055110466, ISSN: 1932-6203, DOI: 10.1371/journal.pone.0015544**
- **CHRISTOPHE E REDON ET AL: "an analysis method for partial-body radiation exposure using-H2AX in non-human primate lymphocytes", RADIATION MEASUREMENTS, ELSEVIER, AMSTERDAM, NL, vol. 46, no. 9, 25 février 2011 (2011-02-25), pages 877-881, XP028291536, ISSN: 1350-4487, DOI: 10.1016/J.RADMEAS.2011.02.017 [extrait le 2011-03-05]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 997 396 B1

- A. HIRAO ET AL: "Chk2 Is a Tumor Suppressor That Regulates Apoptosis in both an Ataxia Telangiectasia Mutated (ATM)-Dependent and an ATM-Independent Manner", MOLECULAR AND CELLULAR BIOLOGY, vol. 22, no. 18, 15 septembre 2002 (2002-09-15), pages 6521-6532, XP055137354, ISSN: 0270-7306, DOI: 10.1128/MCB.22.18.6521-6532.2002
- SHIYU SONG ET AL: "Different Responses of Epidermal and Hair Follicular Cells to Radiation Correlate with Distinct Patterns of p53 and p21 Induction", THE AMERICAN JOURNAL OF PATHOLOGY, vol. 155, no. 4, 1 octobre 1999 (1999-10-01), pages 1121-1127, XP055137366, ISSN: 0002-9440, DOI: 10.1016/S0002-9440(10)65215-7
- K. E. GURLEY ET AL: "Ataxia-Telangiectasia Mutated Is Not Required for p53 Induction and Apoptosis in Irradiated Epithelial Tissues", MOLECULAR CANCER RESEARCH, vol. 5, no. 12, 1 décembre 2007 (2007-12-01), pages 1312-1318, XP055137351, ISSN: 1541-7786, DOI: 10.1158/1541-7786.MCR-07-0223
- C. E. REDON ET AL: "Histone H2AX and Poly(ADP-Ribose) as Clinical Pharmacodynamic Biomarkers", CLINICAL CANCER RESEARCH, vol. 16, no. 18, 7 septembre 2010 (2010-09-07), pages 4532-4542, XP055110503, ISSN: 1078-0432, DOI: 10.1158/1078-0432.CCR-10-0523
- KAI ROTHKAMM ET AL: "[gamma]-H2AX as protein biomarker for radiation exposure", ANN IST SUPER SANITÀ, vol. 45, 1 janvier 2009 (2009-01-01), pages 265-271, XP055110720,
- JENNIFER S DICKEY ET AL: "H2AX: functional roles and potential applications", CHROMOSOMA ; BIOLOGY OF THE NUCLEUS, SPRINGER, BERLIN, DE, vol. 118, no. 6, 26 août 2009 (2009-08-26) , pages 683-692, XP019760098, ISSN: 1432-0886, DOI: 10.1007/S00412-009-0234-4
- KAI ROTHKAMM ET AL: "In situ Biological Dose Mapping Estimates the Radiation Burden Delivered to 'Spared' Tissue between Synchrotron X-Ray Microbeam Radiotherapy Tracks", PLOS ONE, vol. 7, no. 1, 6 janvier 2012 (2012-01-06) , page e29853, XP055110487, DOI: 10.1371/journal.pone.0029853
- BOTCHKAREV V A ET AL: "P53 involvement in the control of murine hair follicle regression", AMERICAN JOURNAL OF PATHOLOGY; [10640], ELSEVIER INC, US, vol. 158, no. 6, 1 juin 2001 (2001-06-01), pages 1913-1919, XP002996833, ISSN: 0002-9440
- Souvik Ghatak ET AL: "A Simple Method of Genomic DNA Extraction from Human Samples for PCR-RFLP Analysis", JOURNAL OF BIOMOLECULAR TECHNIQUES, 1 December 2013 (2013-12-01), page jbt.13-2404-001, XP055402041, US ISSN: 1524-0215, DOI: 10.7171/jbt.13-2404-001

**Description**

DOMAINE TECHNIQUE

La présente invention concerne un procédé d'évaluation par biodosimétrie de la dose d'irradiation reçue par un individu ayant été soumis à un rayonnement ionisant.

ETAT DE L'ART

Dans le cas d'irradiations de type électromagnétique, lors d'un accident ou d'un attentat par exemple, il existe différents moyens de déterminer la dose reçue par un individu (dosimétrie), notamment au travers d'examens biologiques en laboratoire.

On peut, par exemple, mesurer des anomalies au niveau chromosomique. Cette méthode nécessite la mise en culture de cellules prélevées sur l'individu, des étalements chromosomiques et d'autres manipulations de grande technicité. Il est également possible de mesurer l'apoptose des lymphocytes des cellules prélevées. Cette méthode semble moins onéreuse, mais nécessite la mise en culture des cellules et une interprétation extrêmement délicate des résultats. D'autres méthodes basées sur le tri cellulaire ont également été mises au point, en particulier utilisant la méthode dite FACS (pour *fluorescence activated cell sorting*) sur des cellules sanguines. Cette méthode d'analyse intracellulaire permet de mesurer le niveau de marqueurs biologiques (foci) qui est proportionnel au niveau d'irradiation reçu par l'individu.

Ces approches restent toutefois des approches de laboratoire complexes et longues à mettre en oeuvre. D'une manière générale ces procédés de laboratoire sont longs, complexes et coûteux et ne peuvent pas être envisagés pour des tests sur le terrain ou dans le cadre de tests à grande échelle pour un grand nombre de personnes, que ce soit dans le cadre d'un tri de personnes ou de biodosimétrie. De plus, il s'agit de tests de biodosimétrie globale qui renseignent sur le niveau d'irradiation global d'un individu. Ces tests ne sont pas efficaces dans le cas d'une irradiation hétérogène l'individu n'ayant pas reçu la même dose d'irradiation sur l'ensemble du corps. Ceci est dû au fait que ces tests sont essentiellement basés sur des mesures réalisées sur des cellules du sang qui, comme on le sait, circule dans tout le corps d'un individu. Ainsi lors d'irradiations partielles et/ou à faible dose (inférieure à 1 Gy), la mesure est diluée et la dose d'irradiation mesurée est donc sous estimée. Il est en outre impossible d'identifier la partie du corps irradiée.

Dans le cas d'exposition aux neutrons, il existe des tests pouvant être mis en oeuvre sur le terrain qui permettent d'estimer les doses de neutrons reçues. Il est ainsi possible de mesurer le taux de phosphore 32 dans les cheveux ou de sodium 24 dans le sang. Toutefois, là encore, il s'agit de tests de dosimétrie globale.

Toutes ces méthodes sont des tests de dosimétrie globale qui ne permettent pas d'identifier précisément la ou les partie(s) du corps irradiée(s). Pour envisager une biodosimétrie d'une région spécifique du corps d'un individu irradié, il est actuellement nécessaire d'utiliser une méthode invasive consistant à réaliser des biopsies de peaux de la zone concernée. Mais, il n'est pas possible de multiplier les biopsies sur l'individu pour rechercher toutes les régions irradiées d'un corps. De plus, les biopsies réalisées créent des blessures qui peuvent être infectées et ne sont pas compatibles avec un environnement contaminé par des particules radioactives. En effet, les conditions d'asepsie de prélèvement, ainsi que les produits très corrosifs utilisés pour la décontamination des personnes interdisent ce genre de pratique sur le terrain. Enfin, la multiplication des prélèvements complexifie d'autant la mise en oeuvre de techniques de laboratoire et accroît encore le prix du test dans le cas d'un grand nombre d'individus susceptibles d'avoir été irradiés.

On peut également citer des méthodes de mesure des cassures double brin de l'ADN des cellules d'un individu irradié. En effet, il est connu qu'une des conséquences cellulaires majeures des radiations ionisantes est les cassures de l'ADN et plus particulièrement les cassures double brin (CDBs). Il a ainsi été montré que la proportion de cassures double brin de l'ADN chez un individu est directement proportionnelle à la dose d'irradiation reçue par cet individu. La mesure de ces cassures permet donc de déterminer la dose d'irradiation reçue par l'individu.

Les cassures double brin de l'ADN induisent rapidement et de manière coordonnée une série de réponses cellulaires, dont l'activation de systèmes protéiques et enzymatiques pour signaler et réparer les dommages à l'ADN. Cette réponse fait notamment intervenir les protéines du système ATM, appelé aussi cascade ATM. Lors de la monopolisation des systèmes de réponse de la cellule à la suite de cassures double brin, certaines protéines du système ATM telles que Chk2, NBS1, ATM, BRCA1, 53BP1, P53, KU70, etc. sont phosphorylées alors que d'autres sont déphosphorylées. Une des protéines phosphorylées de manière précoce en réponse aux cassures double brin est l'histone H2AX. Cette histone est phosphorylée en $\gamma$-H2AX (Rogakou et al. (1998) J. biol. Chem. 273: 5858-5868; Paull et al. (2000) Curr. Biol. 10:886-895). L'accumulation de $\gamma$-H2AX au niveau des cassures double brin peut être détectée en microscopie d'immunofluorescence, il s'agit de foci $\gamma$-H2AX ou foci détectés par la méthode FACS précitée. Le nombre de foci dans une cellule, est corrélé au nombre de cassures double brin. Il est donc possible de réaliser une biodosimétrie en mesurant le nombre de foci dans les cellules (mesure intracellulaire). Néanmoins, cette approche est très longue, complexe et coûteuse à mettre en oeuvre. De plus, cette méthode ne prend pas en compte les autres protéines du système ATM tels que Chk2, NBS1, ATM, BRCA1, 53BP1, P53, KU70, etc. De plus, aucune des méthodes décrites ne permet de déterminer la dose d'irradiation reçue et le moment auquel elle a eu lieu. C'est notamment le cas dans les documents « The Use of Gamma-H2AX as a Biodosimeter for Total-Body Radiation Exposure in Non-Human Primates » de Christophe E. Redon et al., PLOS ONE, vol. 5, no. 11, 23 novembre 2010, page e15544, et « Qγ-H2AX, an analysis method for partial-body radiation exposure using γ-H2AX in non-human primate lymphocytes » de Christophe E. Redon et al., Radiation Measurements, ELSEVIER, Amsterdam, NL, vol. 46, no. 9, 25 février 2011, pages 877-881.

Il existe donc un réel besoin d'un procédé qui puisse être mis en oeuvre rapidement et particulièrement adapté mais non exclusivement au terrain pour identifier, trier et le cas échéant doser le niveau d'irradiation de personnes irradiées dans le cadre d'un accident ou d'un attentat impliquant des irradiations de type ionisant.

La présente invention a notamment pour but d'apporter une solution simple, efficace et économique à au moins une partie des problèmes précités.

EXPOSE DE L'INVENTION

L'invention propose un procédé d'évaluation par biodosimétrie de la dose d'irradiation reçue par un individu ayant été soumis à un rayonnement ionisant, comprenant les étapes consistant à :

a) prélever, dans au moins une zone du corps de l'individu, des bulbes ou des follicules pileux de poils et/ou de cheveux de l'individu,

b) extraire des protéines de cellules de ces bulbes ou follicules pileux prélevés, ces protéines comprenant des protéines du système ATM ayant subies une phosphorylation et/ou un acétylation induite par le rayonnement ionisant,

c) analyser au moins deux types de protéines extraites, et interpréter les résultats d'analyse afin de déterminer la dose d'irradiation reçue dans la ou chaque zone de prélèvement, les protéines phosphorylées et/ou acétylées du système ATM étant destinées à produire des signaux lors de l'analyse, dont une grandeur telle qu'une intensité ou un ratio d'intensités permet d'évaluer cette dose d'irradiation,

ledit procédé étant caractérisé en ce que lesdits au moins deux types de protéines présentent des cinétiques de phosphorylation/déphosphorylation différentes, et en ce que l'étape c) d'analyse et d'interprétation comprend les sous-étapes consistant à :

- soumettre chaque type de protéines à un marquage, la forme phosphorylée de chaque type de protéines pouvant éventuellement avoir un marquage spécifique,
- soumettre les protéines à une analyse dans laquelle au moins un signal induit par le marquage du type de protéines est étudié,
- comparer une grandeur ou un paramètre, telle que l'intensité, de ce signal à une courbe d'étalonnage représentant l'évolution de cette grandeur en fonction d'une dose d'irradiation reçue par un individu, et
- en déduire la dose d'irradiation reçue par l'individu, dans la zone de prélèvement.

Dans la présente demande, on entend par biodosimétrie, la détermination quantitative de la dose absorbée par une cellule, un organisme ou un individu suite à l'exposition à des rayonnements ionisants. La biodosimétrie est ici extracellulaire, c'est-à-dire que l'analyse de la mesure ne se fait pas à l'intérieur de la cellule (par opposition à la méthode intracellulaire FACS de la technique antérieure), mais directement sur un mélange de protéines obtenu après destruction des membranes plasmique et nucléaire de la cellule (lyse cellulaire). La dose d'irradiation reçue par un individu est en général exprimée en Gray (Gy).

Dans la présente demande, les termes et expressions « poils », « cheveux », « poils et/ou cheveux », désignent indifféremment des poils et/ou des cheveux. Les termes et expressions « bulbes », « follicules pileux », « bulbes et/ou follicules pileux », désignent indifféremment des follicules pileux et/ou des bulbes provenant de poils et/ou cheveux.

On entend par « extraire des protéines », le fait de rendre accessibles des protéines à des anticorps, des marqueurs ou autres à l'extérieur de la cellule.

L'irradiation désigne l'exposition, volontaire ou accidentelle, d'une cellule, d'un organisme, d'une substance, d'un corps, à des rayonnements, en particulier à des rayonnements ionisants. Un rayonnement ionisant est un rayonnement capable de déposer assez d'énergie dans la matière qu'il traverse pour créer une ionisation. Les propriétés des rayonnements ionisants dépendent en particulier de la nature des particules constitutives du rayonnement ainsi que de leur énergie. Ils sont de natures et de sources variées, tels que certains rayonnements ultraviolets, les rayons X, les neutrons, les sources radioactives, les rayonnements gamma, alpha, beta, etc. Un rayonnement ionisant atteignant un organisme vivant endommage ses constituants cellulaires, tels que l'ADN. A faible dose comme à fortes doses, les rayonnements ionisants créent des cassures double brin proportionnellement à la dose reçue. Des mécanismes intracellulaires peuvent permettre de réparer les lésions produites. En revanche, en cas d'exposition à de fortes doses, ces mécanismes sont dépassés et des cassures double brin de l'ADN ne sont pas réparées conduisant le plus souvent à une mort cellulaire par apoptose.

Le système ATM ou cascade ATM est un mécanisme intracellulaire dans lequel certaines protéines subissent une ou plusieurs phosphorylations, acétylations, déphosphorylations, et/ou déacétylations, en réponse à une cassure double brin dans l'ADN. En effet, suite à une cassure double brin de l'ADN, la protéine dimérique ATM est phosphorylée entraînant la séparation du dimère en deux monomères qui peuvent alors se fixer sur des protéines cibles. Des réactions en chaîne de phosphorylation/déphosphorylation de divers substrats tels que l'histone H2AX et les protéines 53BP1, BRCA1, Chk1 et Chk2, vont induire l'arrêt du cycle cellulaire et l'activation des points de contrôle qui vont permettre la réparation de l'ADN endommagé ou l'apoptose des cellules si l'ADN est irréparable.

La phosphorylation d'une protéine est l'addition d'un groupe phosphoryle $PO_3^{2-}$ (appelé plus couramment groupe phosphate) qui est transféré sur une protéine, la déphosphorylation, a contrario, est la perte d'un groupe phosphoryle $PO_3^{2-}$. Dans les deux cas, il s'agit généralement de mécanismes de régulation fréquents. Ainsi, de nombreux enzymes et récepteurs sont mis en position « actif » ou « non actif » par une phosphorylation ou une déphosphorylation.

De même, l'acétylation d'une protéine est le transfert d'un groupe acétyle ($-C(O)CH_3$) sur une protéine. Elle permet généralement l'activation ou l'inhibition d'enzymes.

Comme cela sera décrit plus en détail dans ce qui suit, l'analyse des résultats peut consister notamment à quantifier l'intensité de signaux générés par les protéines phosphorylées et/ou acétylées, de façon à en déduire la dose d'irradiation reçue par l'individu. En effet, plus la dose d'irradiation reçue par un individu est importante, plus le nombre de protéines phosphorylées/acétylées est important, et plus l'intensité des signaux générés pas ces protéines est important lors de l'analyse.

Les étapes du procédé selon l'invention sont relativement simples à mettre en oeuvre ce qui rend le procédé applicable particulièrement mais non exclusivement sur le terrain, par exemple sur le lieu ou à proximité du lieu où la source d'irradiation est située. Ce procédé peut être aussi applicable en milieu médical, notamment après ou avant un examen et/ou un traitement aux rayons X. Ceci est rendu possible par la nature des prélèvements effectués sur l'individu. Ces prélèvements n'affectent pas la santé de l'individu et sont moins douloureux que des biopsies. Ils permettent d'avoir accès à des cellules biologiques situées sur des zones particulières du corps de l'individu. L'invention permet donc de réaliser une mesure locale et rapide d'irradiation (mesure de la dose d'irradiation reçue au niveau d'une zone déterminée du corps de l'individu), tandis que les techniques antérieures ne permettent qu'une mesure globale d'irradiation (mesure de la dose d'irradiation reçue par l'individu dans son ensemble). Les prélèvements peuvent en outre être effectués simultanément sur un grand nombre d'individus et sur une durée relativement courte. La durée de mise en oeuvre du procédé peut être de l'ordre de 20 min par individu, par exemple et d'une manière générale peut être inférieur à 1h. Ce sont plus particulièrement les étapes b) et c) du procédé qui peuvent être mises en oeuvre rapidement. Dans le cas où des prélèvements sont réalisés sur plusieurs zones différentes du corps d'un individu, le procédé permet de déterminer la dose d'irradiation reçue pour chaque zone et ainsi d'établir une cartographie corporelle d'irradiation de l'individu. L'irradiation reçue est de préférence un rayonnement gamma.

La présente invention permet ainsi de déterminer si un individu a été irradié, ainsi que le niveau et l'étendue de l'irradiation de cet individu et éventuellement de déterminer le moment de l'irradiation. Le procédé permet d'avoir un résultat rapide de façon à pouvoir classer rapidement les individus testés selon leur niveau d'irradiation.

De façon avantageuse, le procédé peut être appliqué à plusieurs individus, dans un lieu habituellement non équipé d'appareils de laboratoire. Il peut également comprendre une étape préliminaire d'identification du ou de chaque individu.

Selon l'invention, lesdits au moins deux types de protéines analysées dans l'étape c) présentent des cinétiques de phosphorylation/déphosphorylation différentes. Autrement dit, lors de l'analyse, les signaux correspondants aux deux types de protéines ont des évolutions différentes, ce qui permet notamment de dater l'irradiation. Lors d'une observation ponctuelle des signaux relatifs aux deux protéines, le niveau de chaque signal permet, grâce à une courbe de calibration, par exemple en trois dimensions, définissant la réponse en fonction des doses et du temps après l'exposition, de combiner l'information provenant des deux protéines pour définir la dose reçue par rapport au temps écoulé après l'irradiation. Dans une observation cinétique des signaux des deux protéines, par exemple au travers de plusieurs prélèvements, chaque courbe de signal comprend deux parties, une première partie qui croît (dans laquelle l'intensité augmente du fait de l'augmentation du taux de phosphorylation des protéines) et une deuxième partie qui décroît (l'intensité diminue du fait de la déphosphorylation des protéines en réponse à la réparation de l'ADN). Le signal correspondant à la protéine H2AX est différent de ceux des autres protéines du système ATM et en particulier de celui de la protéine Ku70. Le signal de la protéine H2AX comprend en général une première partie qui croît rapidement au contraire de celui de la protéine Ku70 qui comprend une première partie qui croît plus lentement. A un instant t, même si l'intensité du signal d'un des types de protéines diminue, l'intensité du signal de l'autre type est prise en compte pour augmenter la précision de la mesure. L'évolution du signal et sa vitesse d'évolution (derivée des courbes ou accroissement fini des courbes) donnent les informations correctrices pour estimer la dose en fonction de la radiosensibilité individuelle. La combinaison des informations d'au moins deux marqueurs permet de définir d'une part en observation ponctuelle la dose reçue et le retard de la mesure après l'observation, et d'autre part en observation dynamique le niveau de radiosensibilité.

Dans un mode préféré de réalisation de l'invention, les protéines analysées à l'étape c) sont au moins H2AX et Ku70. L'analyse de la forme phosphorylée de Ku70 est avantageuse car les inventeurs ont constaté que le signal correspondant est plus persistant que celui de γ-H2AX et se renforce pendant une durée plus longue du fait que la première partie de sa courbe croît lentement au contraire de γ-H2AX.

Dans un mode de réalisation préférentiel, les protéines sont immobilisées sur un support à l'étape c), les protéines étant alors fixées soit directement, c'est à dire par des interactions avec le support, soit indirectement audit support. Cette fixation indirecte peut être réalisée de manière spécifique (par l'intermédiaire d'un anticorps, un heptamère ou toute autre molécule reconnaissant spécifiquement la ou les protéines d'intérêt) ou aspécifique (par exemple au travers de molécules ADN fixées au support). La fixation des protéines sur un support permet de faciliter et d'accélérer l'analyse, et permet donc d'obtenir des résultats relativement rapidement, par exemple en moins d'une heure. La fixation peut être réalisée par voie chimique, par exemple par liaison covalente. La fixation peut se faire sur un support quelconque, tel qu'une particule (micrométrique, sub-micrométrique ou nanométrique), une membrane (PVDF ou nitrocellulose, ou toute autre membrane pouvant capturer les protéines de manière directe ou indirecte), ou un filtre permettant de capturer les protéines. Dans un mode de réalisation préférentiel, les supports comportent des phases diamants, dépôt de diamant ou particules de diamant permettant de capturer les protéines directement ou indirectement. Dans un mode de réalisation encore plus préférentiel, ces phases diamant seront hydrogénées de manière à obtenir des carbone C-H capables d'établir des liaisons covalentes à travers un carbamide.

Dans certains modes de réalisations, le support peut comporter des fragments d'acide nucléique ou analogue d'acide nucléique complémentaire de fragments d'accroche acide nucléique ou analogue, fixés sur les molécules capables de reconnaître spécifiquement les protéines d'intérêts qui, en s'hybridant avec lesdits fragments d'accroche, permettent la fixation des molécules capables de reconnaître spécifiquement les protéines d'intérêt sur le support.

Dans certains modes de réalisation, les supports particulaires sont paramagnétiques ou sensibles à un champ magnétique.

Dans un mode de réalisation particulièrement préféré, l'étape c) d'analyse est effectuée directement sur le support sur lequel sont fixées les protéines extraites à l'étape b) ce qui permet une mise en oeuvre du procédé très rapide.

Dans un mode de réalisation particulier, les follicules des poils sont prélevés dans plusieurs zones distinctes du corps, de façon à déterminer la dose d'irradiation reçue par chaque zone.

Dans un mode de réalisation particulier du procédé selon l'invention, l'étape a) de prélèvement comprend les sous-étapes consistant à :

- appliquer un patch sur la ou chaque zone, et le retirer pour arracher des poils avec leurs follicules dans cette zone, et
- introduire le patch et les poils avec leurs follicules dans un puits de réaction.

Le patch peut comprendre une cire, une colle, une glue ou de toute autre matière collante permettant de réaliser une épilation. Après application du patch sur la zone de prélèvement, le retrait du patch entraîne l'arrachage des poils avec leurs follicules.

Dans la présente demande, on entend par puits de réaction ou puits, un récipient dans lequel les protéines d'intérêt sont extraites et peuvent être isolées. Un puits de réaction a typiquement un volume de quelques microlitres à quelques millilitres. De façon préférentielle, le puits de réaction peut être le puits d'une plaque multi-puits, telle qu'une plaque de 6, 12, 24, 48 ou 96 puits. Dans le cas d'un établissement d'une cartographie corporelle d'irradiation d'un individu, chaque prélèvement est introduit dans un puits donné d'une plaque multi-puits. Chaque puits est de préférence pourvu d'un moyen de repérage permettant de connaître à quel prélèvement il est associé.

Selon un premier exemple de réalisation de l'invention, le patch est fixé à une extrémité d'un piston mobile dans un tube entre une position avancée et une position reculée. En position avancée, le patch s'étend au moins en partie à l'extérieur du tube et peut être appliqué par l'intermédiaire du piston sur la zone à épiler. En position reculée, le patch est logé dans le tube, le retrait du patch de la zone provoque l'arrachage des poils avec leurs follicules. Le recul du piston dans le tube provoque l'introduction des follicules des poils attachés au patch, dans le tube.

Selon un second exemple de réalisation de l'invention, le patch est formé par une bandelette flexible qui est fixée par une extrémité à une tige portée par un bouchon, la bandelette étant destinée à être enroulée autour de la tige avant son introduction dans le puits de réaction. Les poils sont de préférence situés à l'extérieur de la bandelette après enroulement.

Dans un troisième exemple de réalisation, le patch est fixé sur une membrane de matière plastique flexible qui permet une application sur une plus grande surface corporelle et donc l'obtention d'un prélèvement avec davantage de follicules. La membrane flexible peut être destinée à fermer l'ouverture des puits de réaction, la cire ou glue du patch servant de colle pour la fermeture de ces puits. La cire ou la glue du patch a de préférence une épaisseur supérieure à celle des poils de façon à ce que le patch puisse être collé sans que les poils ne gênent le collage ainsi que son étanchéité.

Dans le premier exemple de réalisation, le recul du piston dans le tube entraîne le repliement des follicules pileux vers l'intérieur du tube. Le repliement facilite l'introduction des poils prélevés avec leurs follicules pileux dans le puits de réaction. Dans le second exemple de réalisation, le patch est enroulé autour d'une tige pour pouvoir être introduit facilement dans le puits de réaction. Il est possible de maintenir le patch enroulé par un adhésif disposé sur la tige. Dans le troisième exemple, le patch est directement appliqué sur l'ouverture d'un puits de réaction de manière à fermer cette ouverture avec le patch formant ainsi un film de fermeture, la glue utilisée servant de colle de fermeture. Les trois exemples de réalisation permettent un gain de temps non négligeable lors de tests réalisés sur un grand nombre d'individus et sur un grand nombre de zones du corps de chaque individu. Ainsi, pour un individu donné, il est possible de faire en moins d'une heure un test de biodosimétrie précis et de connaître la dose d'irradiation reçue pour chaque zone du corps où un prélèvement a été effectué.

De façon avantageuse, l'introduction du patch dans le puits est réalisée en emboîtant une extrémité ouverte du tube précité (du premier exemple de réalisation) dans le puits ou en insérant la tige et la bandelette (du second exemple de réalisation) dans le puits puis en fermant ce puits avec le bouchon portant la tige.

Dans un mode de réalisation particulier, le procédé selon l'invention comprend, en outre, les étapes consistant à :

- prélever du sang de l'individu, par exemple à l'extrémité d'un doigt,
- récupérer des cellules du sang prélevé, à l'exception des globules rouges, et extraire des protéines de ces cellules, ces protéines comprenant des protéines du système ATM ayant subies une phosphorylation et/ou une acétylation induite par le rayonnement ionisant.

Dans la présente demande, les « cellules » récupérées dans le sang prélevé sont les cellules sanguines dont sont exclus les globules rouges.

L'analyse des cellules sanguines permet de déterminer le taux de lésion de l'ADN génomique des lymphocytes. Il s'agit d'une mesure globale de la dose d'irradiation reçue par un individu.

De préférence, ces étapes précèdent l'étape a) ou sont réalisées simultanément aux étapes a) et b). Il est ainsi possible de réaliser simultanément ou quasi-simultanément, le prélèvement de sang et des poils. Il est également possible de réaliser simultanément ou quasi-simultanément les étapes d'extraction des protéines des cellules sanguines et des follicules pileux. Ceci permet un gain de temps non négligeable.

Il est cependant tout à fait envisageable de réaliser les étapes précitées relatives aux cellules sanguines à tout moment dans le procédé décrit précédemment.

Dans un mode de réalisation particulier du procédé selon l'invention, l'étape de prélèvement du sang est précédée d'une étape préliminaire consistant à appliquer une solution contenant de l'alcool et de l'héparine (ou n'importe quel mélange contenant un antiseptique et un anticoagulant) sur la zone de prélèvement, cette étape de prélèvement pouvant être suivie d'une étape supplémentaire consistant à appliquer une solution contenant de l'alcool et du chlorure de calcium (ou n'importe quel mélange contenant un antiseptique et un coagulant) sur la zone de prélèvement.

L'antiseptique peut être sélectionné parmi les alcools, tels que l'éthanol ou l'isopropanol ou un mélange de désinfectants ou antiseptiques, de préférence il s'agira d'un alcool.

L'anticoagulant peut être de l'héparine.

Dans un mode de réalisation particulier, l'anticoagulant et l'antiseptique sont imprégnés dans une lingette jetable rangée dans une pochette individuelle fermée de manière hermétique et d'ouverture rapide.

De même, le coagulant et l'antiseptique peuvent être imprégnés dans une lingette jetable rangée dans une pochette individuelle fermée de manière hermétique et d'ouverture rapide.

Le coagulant permet la coagulation sanguine après le prélèvement. Celui-ci évite une éventuelle hémorragie, notamment dans le cas d'un individu hémophile. De préférence il s'agit du chlorure de calcium.

Selon un mode de réalisation, le prélèvement de sang est réalisé au moyen d'une lancette destinée à percer la peau de l'individu. Cette lancette comporte une lame éjectable et comprend de préférence une chambre de stockage d'une solution d'alcool et d'héparine (ou de n'importe quel mélange contenant un antiseptique et un anticoagulant tel que oxalate, citrate, EDTA, héparine, etc.), qui est destinée à être libérer au moment de l'éjection de la lame.

La lame éjectable de la lancette est éjectée et transperce la peau de l'individu dans le but de faire un prélèvement de sang. De préférence, la piqûre sera réalisée au bout d'un doigt. Cette lancette diminue les risques de contamination que l'on pourrait avoir par prélèvement de sang avec une seringue, par exemple, car le prélèvement est moins invasif et la blessure plus petite. De plus, une telle lancette permet à l'individu de faire lui-même le prélèvement et ainsi de restreindre la durée du prélèvement. Il est possible de prévoir dans la lancette une chambre de stockage de la solution contenant l'antiseptique et/ou l'anticoagulant, de telle sorte que, lorsque la lame, initialement à l'intérieur de la lancette, est éjectée, celle-ci transperce la chambre et libère son contenu. De préférence, cette chambre comprendra une solution d'héparine pour empêcher la coagulation du sang et ainsi prélever la quantité de sang suffisante pour réaliser le test.

Il peut s'agir d'une lancette jetable, par exemple une lancette destinée aux diabétiques.

Selon un mode de réalisation du procédé selon l'invention comprenant une étape de prélèvement de sang, l'étape de récupération des cellules consiste à :

- introduire le sang prélevé dans un tube contenant une suspension de billes magnétiques fonctionnalisées avec des anticorps contre des antigènes de groupe sanguin, et éventuellement de l'héparine (ou n'importe quel anticoagulant),
- séparer les billes d'un liquide résiduel au moyen d'un champ magnétique, le liquide résiduel comportant les cellules du sang, à l'exception des globules rouges, et transférer le liquide résiduel dans un puits de réaction.

Dans la présente demande, l'expression « liquide résiduel » désigne un liquide comprenant notamment du plasma avec des lymphocytes et des plaquettes, et en particulier les cellules faisant l'objet du test.

Les billes magnétiques fonctionnalisées avec des anticorps contre des antigènes de groupe sanguin permettent de séparer les globules rouges des cellules d'intérêt du sang contenues dans le liquide résiduel, les globules rouges étant susceptibles de générer des signaux indésirables rendant l'interprétation des mesures compliquées. Les billes magnétiques pourvues d'anticorps se fixent aux globules rouges pour former des caillots. Ces caillots sont précipités et retenus à l'aide d'un aimant lors du transfert du liquide résiduel dans le puits de réaction. Un anticoagulant tel que de l'héparine peut être ajouté au sang prélevé et/ou au puits de réaction.

Dans un autre mode de réalisation, les billes comportent des anticorps dirigés contre les lymphocytes exclusivement, de manière à capturer que ces derniers et à les séparer des autres éléments figurés du sang. L'extraction des protéines étant alors faite sur les cellules lymphocytaires précipitées par les billes

Selon un mode de réalisation du procédé selon l'invention, l'extraction des protéines comprend les sous-étapes consistant à :

- mettre en contact ou mélanger les follicules pileux prélevés et/ou le liquide résiduel récupéré avec une solution de lyse cellulaire, ou
- mettre en contact ou mélanger les cellules avec une solution non dénaturante, de préférence exempte de phosphore, puis soumettre l'ensemble à une énergie électromagnétique ou mécanique, tel que micro-onde ou par ultrason, destinée à provoquer la lyse des cellules.

La lyse cellulaire permet la destruction de la membrane plasmique et nucléaire de la cellule et l'extraction des protéines présentes à l'intérieur de celle-ci. Elle peut être réalisée par voie chimique, par voie mécanique ou par l'intermédiaire d'ondes (micro-ondes, ultrasons, etc.). Dans le cas de la voie chimique une solution de lyse cellulaire est introduite dans le puits de réaction. La solution de lyse cellulaire peut comprendre soit une solution hypotonique (concentration en NaCl < 150mM), soit une solution hypertonique (concentration en NaCl > 150mM) avec une addition de détergents enzymatiques (0,5-1% NP40, 0,1-1% SDS, triton, ripa, etc). Dans le cas d'une lyse cellulaire mécanique ou par l'intermédiaire d'ondes, les cellules peuvent être mises en contact ou mélanger avec une solution non-dénaturante. Elles sont ensuite soumises à une lyse mécanique provoquée par exemple par une presse, des billes de verre ou magnétiques (mises en mouvement par un champ magnétique), par ultrason etc. Dans un mode de réalisation préférentiel, la lyse est réalisée par l'intermédiaire d'ondes par exemple micro-ondes. De préférence, la solution non dénaturante est exempte de phosphore pour ne pas perturber par la suite l'interprétation des résultats, en particulier lors d'une analyse LIBS. Il peut s'agir d'une solution de TBS (Tris buffer saline) ou de TBE (Tris buffer EDTA). La lyse cellulaire est de préférence réalisé par micro-onde, ce qui permet de ne pas modifier l'état de phosphorylation des protéines.

De façon avantageuse, les différents prélèvements sont répartis dans différents puits de réaction d'une plaque multi-puits. Dans le cas d'un grand nombre de prélèvements, cela permet de faciliter les différentes étapes suivant le prélèvement, notamment de les automatiser. Une plaque multi-puits peut être affectée à chaque individu testé. Les puits de cette plaque comprennent des prélèvements de plusieurs zones distinctes du corps de l'individu.

Dans un mode de réalisation utilisant une plaque multi-puits, le fond des puits peut être formé par une membrane, éventuellement filtrante, sur laquelle les protéines sont destinées à se déposer ou à se fixer. Elles peuvent se fixer sur le fond directement ou par l'intermédiaire d'anticorps spécifiques préalablement fixés sur le fond ou à des particules. Dans un mode de réalisation préférentiel, le fond des puits est amovible.

Dans un mode de réalisation de l'invention, des marqueurs sont introduits dans les puits de réaction. Ces marqueurs sont des marqueurs fluorescents, colorimétriques, chimioluminescents, et/ou des anticorps et sont destinés à se lier à des protéines phosphorylées et/ou non phosphorylées.

Dans un mode de réalisation de l'invention, des anticorps destinés à fixer les protéines recherchées sont fixés à des particules qui sont introduites dans les puits de réaction. Dans un mode de réalisation encore plus particulier, chaque type d'anticorps est fixé à une particule de taille donnée et qui est différente des tailles des particules portant les autres types de marqueurs.

Dans un mode de réalisation de l'invention, le fond de chaque puits de réaction comprend un test d'immunochromatographie. Il peut s'agir par exemple d'une bandelette de nitrocellulose permettant la migration d'une phase mobile. La phase mobile comprend des complexes anticorps marqué/protéine cible, des protéines, et des anticorps marqués. Les anticorps sont marqués, par exemple, par des particules ou billes d'or. Dans ce mode de réalisation, la bandelette comprend dans une zone de dépôt des anticorps marqués dirigés contre l'épitope phosphorylé d'au moins une protéine cible de la phase mobile. Ces anticorps sont non fixés à la bandelette et peuvent être lyophilisés. La bandelette comprend également au moins une zone comportant des anticorps dirigés contre un épitope non phosphorylé d'au moins une protéine cible. Ces anticorps sont fixés à la bandelette et forment la phase fixe. La bandelette peut également comporter au moins une zone dirigée contre les anticorps de la phase mobile ou contre le marquage de ces anticorps. Lorsque l'échantillon de protéines extraites est déposé sur la zone de dépôt de la bandelette, les protéines cibles se complexent avec les anticorps marqués spécifiques de la phase mobile. L'hydratation de la bandelette permet à la phase mobile (complexes anticorps marqué/protéine cible, protéines et anticorps marqués) de migrer dans la bandelette de nitrocellulose. En passant dans les zones comprenant les phases fixes, les complexes anticorps marqué/protéine cible phosphorylée et les protéines cibles non phosphorylées sont retenues au niveau des anticorps de la phase fixe. L'intensité du marquage au niveau des différentes phases fixes indique le taux de protéines phosphorylées. Plusieurs zones successives comprenant une phase fixe différente peuvent être disposées pour retenir différents types de protéines cibles. La dernière zone comprendra une phase fixe capturant les anticorps marqués de la phase mobile qui n'ont pas réagi. Le ratio entre l'intensité du marquage de la phase fixe ayant capturé les protéines cibles et l'intensité du marquage de la phase fixe ayant capturé les anticorps qui n'ont pas réagi permet de déterminer le nombre de cibles.

Dans certains modes de réalisation, une quantité connue d'au moins une protéine cible marquée (protéine référence) est introduite dans l'extrait de protéines à analyser. Cette protéine marquée concurrence la complexation de la protéine cible au niveau de la phase fixe correspondante pour obtenir un signal de l'échantillon (léchantillon). Une même quantité de la protéine cible marquée est introduite dans un autre puits de réaction pour obtenir un signal de référence (Iref). La comparaison des intensités des signaux Iref et I obtenus entre le puits comprenant la protéine référence seule et le puits comprenant la protéine référence mélangée à l'extrait de protéines à analyser permet de normaliser les mesures de phosphorylation à partir d'un extrait protéique non marqué, tel que le signal retenu est léchantillon/Iref ou IRef/I.

Dans certains modes de réalisation, les anticorps de la phase mobile et des phases fixes peuvent être inversés, c'est-à-dire les anticorps de la phase mobile sont contre les formes non phosphorylées et les anticorps de la phase fixe contre les formes phosphorylées.

Dans certains autres modes de réalisation, les anticorps peuvent être marqués par d'autres marqueurs que des particules d'or, tels que des marqueurs fluorescents, colorimétriques ou chimioluminescents.

De même, les protéines cibles peuvent être marquées de manière différente par des marqueurs fluorescents, colorimétriques ou chimioluminescents.

Selon l'invention, l'étape c) d'analyse et d'interprétation comprend les sous-étapes consistant à :

- soumettre chaque type de protéines à un marquage, la forme phosphorylée de chaque type de protéines pouvant éventuellement avoir un marquage spécifique,
- soumettre les protéines à une analyse dans laquelle au moins un signal induit par le marquage du type de protéines est étudié,
- comparer une grandeur, telle que l'intensité, de ce signal à une courbe d'étalonnage représentant l'évolution de cette grandeur en fonction d'une dose d'irradiation reçue par un individu, et
- en déduire la dose d'irradiation reçue par l'individu, dans la zone de prélèvement.

De façon avantageuse, dans la sous-étape d'analyse précédente, les protéines sont analysées :

- par un procédé LIBS qui permet de quantifier au moins la phosphorylation, chaque type de protéines, dans ses formes phosphorylée et non phosphorylée, pouvant être éventuellement soumis à un marquage, par exemple au bore, ou
- par fluorescence, colorimétrie, ou chimioluminescence, chaque type de protéines ayant été soumis à un marquage fluorescent, colorimétrique ou chimioluminescent.

Une quantité prédéterminée de substance chimio radiomimétique peut être ajoutée aux protéines extraites, on parle alors d'échantillon traité

Une quantité prédéterminée de substance chimio radiomimétique peut être ajoutée aux protéines extraites, le paramètre étudié est alors :

- IP : l'intensité d'au moins un signal correspondant à la forme phosphorylée d'un de chaque type de protéines, et/ou
- IP/I, le ratio entre l'intensité IP et l'intensité I, I étant l'intensité d'au moins un signal correspondant au marquage de ce chaque type de protéines, dans ses formes phosphorylée et non phosphorylée, et/ou

- IP/IPtraité : le ratio entre l'intensité IP et l'intensité IPtraité, IPtraité étant l'intensité d'au moins un signal correspondant à la forme phosphorylée et traité d'un de chaque type de protéines, et/ou
- IPtraité/Itraité : le ratio entre l'intensité IP traité et l'intensité Itraité, Itraité étant l'intensité du signal correspondant au marquage de ce chaque type de protéines traitées, dans ses formes phosphorylée et non phosphorylée, et/ou
- (IP/I) / (IPtraité/Itraité) : le rapport entre le ratio entre les intensités IP et I et le ratio entre les intensités IPtraité et Itraité.

Dans un mode de réalisation de l'invention, l'étape a) consiste à prélever des follicules de poils à au moins deux reprises dans une même zone de prélèvement.

De façon avantageuse, les prélèvements sont effectués à un intervalle de temps $\Delta t$ prédéterminé ou sont réalisés quasi-simultanément et au moins l'un des prélèvements est mis en culture pendant une durée déterminée $\Delta t$.

Les effets de l'irradiation sont immédiats mais déclinent au fil du temps. En effet, le mécanisme de réparation des cassures double brin de l'ADN se met en place dès l'irradiation et de façon différente pour chaque individu. Pour un même individu, le taux de phosphorylation ne sera alors pas le même à l'instant t=0 et à l'instant $\Delta t$. Il est donc utile de connaître la vitesse de réparation des cassures double brin de l'ADN de chaque individu. Ce double prélèvement permet ainsi de connaître la vitesse de réparation des cassures double brin pour chaque individu et de la prendre en compte pour plus de précision dans la mesure de la dose d'irradiation reçue. Cela permet également de connaître la radio-susceptibilité et la variation interindividuelle de chaque individu. La durée $\Delta t$ peut être comprise entre 1 min et 12 h.

La radiosensibilité ou radio-susceptibilité d'un individu correspond à la sensibilité des tissus vivants aux rayonnements ionisants, notamment la vitesse à laquelle l'individu répare son ADN à l'instant où la mesure est faite. Elle varie d'un individu à l'autre, mais également au cours du temps pour un individu donné.

La variation interindividuelle correspond, ici, à la vitesse de réparation de la cassure double brin d'un individu donné.

Dans un mode de réalisation de l'invention comprenant au moins deux prélèvements espacés d'une durée $\Delta t$, la grandeur ou le paramètre étudié est :

- IP : l'intensité d'au moins un signal correspondant à la forme phosphorylée d'un de chaque type de protéines, et/ou
- IP/I : le ratio entre les intensités IP et I, I étant l'intensité d'au moins un signal correspondant au marquage de ce type de protéines, dans ses formes phosphorylée et non phosphorylée, et/ou
- dIP/dt : la variation au cours du temps de l'intensité IP, et/ou
- d(IP/I)/dt : la variation au cours du temps du ratio IP/I.

Dans un mode de réalisation de l'invention comprenant au moins deux prélèvements de follicules de poils, au moins deux des prélèvements sont mélangés à une substance chimio radiomimétique à dose connue. L'un des mélanges est analysé immédiatement et l'autre est analysé après une durée déterminée. La grandeur ou le paramètre étudié est :

- IPtraité : l'intensité d'au moins un signal correspondant à la forme phosphorylée et traité d'un de chaque type de protéines, et/ou
- IPtraité/Itraité : le ratio entre l'intensité IPtraité et l'intensité Itraité, Itraité étant l'intensité du signal correspondant au marquage de ce chaque type de protéines traitées, dans ses formes phosphorylée et non phosphorylée, et/ou
- dIPtraité/dt : la variation au cours du temps de l'intensité IPtraité, et/ou
- d(IPtraité/Itraité)/dt : la variation au cours du temps du ratio IPtraité/Itraité.

La substance chimio radiomimétique génère des cassures double brin de l'ADN. Il est ainsi connu qu'une quantité donnée de substance chimio radiomimétique équivaut à un taux de cassures double brin donné et par conséquent à une dose d'irradiation donnée. Ainsi, de manière similaire au double prélèvement de follicules pileux, les taux de cassures double brin à l'instant t=0 puis après une durée déterminée $\Delta t$ sont connus et il est possible de déterminer la vitesse de réparation des cassures double brin de l'ADN de chaque individu. On gagne alors en précision dans la mesure de la dose d'irradiation reçue. La radio-susceptibilité de chaque individu peut être déterminée par cette méthode. La substance chimio radiomimétique est de préférence la néocarzinostatine (NCS). De préférence, elle sera présente dans le puits de réaction au moment de l'introduction du prélèvement dans ce puits.

Dans certains modes de réalisation, un seul des deux prélèvements est mis en contact avec une dose connue de NCS afin de déterminer le taux de réponse de base d'un individu. En effet, au-delà de la vitesse de réparation de l'ADN, le taux de dommage causé à l'ADN par une même irradiation ionisante varie d'un individu à l'autre. La grandeur ou le paramètre étudié peut être :

- IP : l'intensité d'au moins un signal correspondant à la forme phosphorylée d'un type de protéines,
- IP/I : le ratio entre l'intensité IP et l'intensité I, et/ou
- IP/IPtraité : le ratio entre l'intensité IP et l'intensité IPtraité, IPtraité étant l'intensité d'au moins un signal correspondant à la forme phosphorylée et traité d'un type de protéines, et/ou

- IPtraité/Itraité : le ratio entre l'intensité IP traité et l'intensité Itraité, Itraité étant l'intensité du signal correspondant au marquage de ce type de protéines traitées, dans ses formes phosphorylée et non phosphorylée, et/ou
- (IP/I) / (IPtraité/Itraité) : le rapport entre le ratio entre les intensités IP et I et le ratio entre les intensités IPtraité et Itraité.

D'une manière générale, la grandeur utilisée pour quantifier la dose reçue et/ou la radio-susceptibilité pourra être une combinaison linéaire ou non linéaire des grandeurs précitées.

Ainsi, il est possible de réaliser une cartographie permettant d'évaluer rapidement la dose d'irradiation locale et globale d'un individu irradié en fonction de sa radio-sensibilité.

La dose d'irradiation ou le moment de l'irradiation peut être déterminée au moyen d'une surface répertoriant la variation du taux de phosphorylation d'au moins une protéine en fonction de la dose d'irradiation reçue et du temps de retard de l'observation. La dose d'irradiation ou le moment de l'irradiation peut être déterminée au moyen d'une dérivée ou d'un accroissement fini de ladite surface.

Il est par ailleurs décrit un dispositif de prélèvement de poils d'un individu, particulièrement adapté mais non exclusivement pour la mise en oeuvre du procédé tel que décrit précédemment. Il comprend :

- un patch fixé à une extrémité d'un piston qui est mobile dans un tube depuis une position avancée dans laquelle le patch s'étend au moins en partie à l'extérieur du tube et peut être appliqué par l'intermédiaire du piston sur une zone de prélèvement, et une position reculée dans laquelle le patch est logé dans le tube, le retrait du patch de la zone étant destiné à provoquer l'arrachage des poils avec leur follicules et le recul du piston dans le tube étant destiné à provoquer l'introduction des follicules des poils attachés au patch dans le tube, et/ou
- un patch formé par une bandelette flexible qui est fixée par une extrémité à une tige portée par un bouchon, la bandelette étant destinée à être appliquée sur une zone de prélèvement puis à être extraite de cette zone pour arracher des poils avec leurs follicules de la zone, et la bandelette étant configurée pour être enroulée autour de la tige, et/ou
- un patch comprenant une bandelette flexible d'un matériau synthétique plastique (polymère, etc.) destinée à la fois à être appliquée sur une zone de prélèvement puis à être extraite de cette zone pour arracher des poils avec leurs follicules de la zone, et à être appliquée et collée sur une ouverture d'au moins un puits de réaction en vue de le fermer hermétiquement.

Il est par ailleurs décrit un dispositif de prélèvement de sang d'un individu, particulièrement adapté mais non exclusivement pour la mise en oeuvre du procédé tel que décrit précédemment. Il comprend une lancette destinée à percer la peau de l'individu. Cette lancette comporte une lame éjectable et comprend de préférence une chambre de stockage d'une solution d'alcool et d'héparine (ou de n'importe quel mélange contenant un antiseptique et un anticoagulant), qui est destinée à être libérer au moment de l'éjection de la lame. La chambre est préférentiellement composée d'une capsule en un polymère biologique (naturel, tel que la gélatine, un sucre, de la cellulose, etc.) ou artificiel disposée à l'avant de la lancette rétractée, de sorte que l'éjection de la lancette provoque la rupture de la chambre et la libération de son contenu.

Il est par ailleurs décrit un kit particulièrement adapté mais non exclusivement pour la mise en oeuvre du procédé tel que décrit précédemment. Il comprend au moins un dispositif de prélèvement de poils d'un individu, tel que décrit précédemment par exemple, éventuellement un dispositif de prélèvement de sang d'un individu, tel que décrit précédemment par exemple, et au moins une plaque multi-puits. Les patchs utilisés pour le prélèvement des poils sont destinés à être introduits dans des puits différents ou à fermer des puits différents d'une plaque multi-puits.

DESCRIPTION DES FIGURES

L'invention sera mieux comprise et d'autres détails, caractéristiques et avantages de l'invention apparaîtront à la lecture de la description suivante faite à titre d'exemple non limitatif et en référence aux dessins annexés, dans lesquels :

- la figure 1 est un organigramme représentant les étapes d'un mode de réalisation du procédé selon l'invention,
- les figures 2 et 3 sont des vues schématiques en coupe axiale d'un dispositif de prélèvement, et représentent respectivement deux positions du piston de ce dispositif,
- les figures 4 et 5 sont des vues schématiques d'un autre dispositif de prélèvement, vu de côté et de dessus, et représentant respectivement deux positions de la bandelette de ce dispositif,
- la figure 6 représente une plaque multi-puits dans les puits de laquelle les dispositifs de prélèvement sont destinés à être emboîtés,
- la figure 7 est une vue schématique en coupe axiale d'un puits de réaction dans lequel un dispositif selon les figures 2 et 3 a été emboîté,

- la figure 8 est une vue schématique en coupe axiale d'un puits de réaction dans lequel un dispositif selon les figures 4 et 5 a été emboîté,
- la figure 9 est une vue schématique en coupe axiale d'un puits de réaction contenant des poils mis en contact avec une solution,
- la figure 10 est une vue schématique d'un dispositif de prélèvement de sang à lame éjectable,
- la figure 11 est une vue schématique en coupe axiale du dispositif de la figure 10,
- les figures 12 et 13 sont des vues schématiques en coupe axiale d'un puits de réaction et représentent des étapes de récupération et de transfert des cellules de sang prélevé,
- la figure 14 représente de manière schématique le transfert du contenu des puits de réaction d'une plaque multi-puits dans les puits de réaction d'une autre plaque multi-puits au moyen d'une pipette multicanaux.
- la figure 15 est une vue schématique en coupe transversale d'une plaque multi-puits à fond amovible,
- la figure 16 est une vue schématique en coupe transversale d'une plaque multi-puits à membrane filtrante qui est posée sur un système d'aspiration du contenu de chaque puits,
- la figure 17 représente de manière schématique des étapes d'extraction, d'isolation, de filtration et de dépôt des protéines sur le fond d'un puits de réaction, du procédé selon l'invention,
- la figure 18 représente de manière schématique des étapes d'extraction, d'isolation, de filtration et de capture des protéines par des anticorps fixés sur le fond d'un puits de réaction, du procédé selon l'invention,
- la figure 19 représente de manière schématique différentes étapes de marquage des protéines dans les puits de réaction,
- la figure 20 représente de manière schématique le fond d'un puits de réaction, comprenant plusieurs zones de fixation d'anticorps spécifiques,
- la figure 21 est une vue schématique d'un appareil d'analyse LIBS,
- La figure 22 est un spectre représentant les résultats d'une analyse LIBS de protéines prélevées,
- la figure 23 est un graphe représentant l'évolution de l'intensité d'un signal LIBS en fonction de la dose d'irradiation reçue par un individu,
- la figure 24 est une courbe comparative entre les méthodes de détection FACS et LIBS,
- la figure 25 représente l'évolution de l'intensité directe de fluorescence en fonction de la dose d'irradiation reçue par un individu,
- la figure 26 est un graphe représentant l'évolution de l'intensité de fluorescence en fonction de la dose d'irradiation reçue par un individu,
- la figure 27 est un graphe représentant l'évolution du taux de cellules apoptotiques d'un mélange en fonction de la quantité de NCS dans ce mélange,
- la figure 28 est un graphe représentant l'évolution du taux de cellules apoptotiques en fonction de la dose d'irradiation reçue par un individu,
- la figure 29 représente l'évolution de la quantité de protéines Ku70 dans des cellules exposées à une irradiation unique de 10Gy à gauche sur le dessin, et à plusieurs irradiations successives de 1Gy, une fois par semaine pendant sept semaines, à droite sur le dessin (NT : protéines non traitées)
- la figure 30 représente la quantité de protéines Ku70 dans des cellules exposées à une irradiation de 10Gy, 30 minutes et 24 heures après l'irradiation (NT : protéines non traitées),
- la figure 31 représente la différence de performance entre des mesures de fluorescence de protéines H2AX de cellules irradiées à 0, 2 et 8GY réalisé par un anticorps (Millipore) et par un anticorps Fab-zipper,
- la figure 32 représente les emplacements de patchs sur un individu, et
- les figures 33a, b et c sont des graphes représentant l'évolution du signal de fluorescence d'un marquage anti Ku70 en FACS pour des extraits de protéines de cellules humaines (ZR75.1) irradiées à 10Gy (gris) ou non (noir), et capturés sur particules de nanodiamants hydrogénés, avec différents mode d'extraction,
- la figure 34 représente un exemple de mise au point de l'extraction des protéines par l'intermédiaire de micro-ondes,
- la figure 35 représente une mise en oeuvre d'un transfert du type *dot blot* d'échantillons irradiés à 0,5Gy sur des membranes, et
- les figures 36 à 41 sont des graphes illustrant un exemple de méthode de quantification selon l'invention.

## DESCRIPTION DETAILLEE

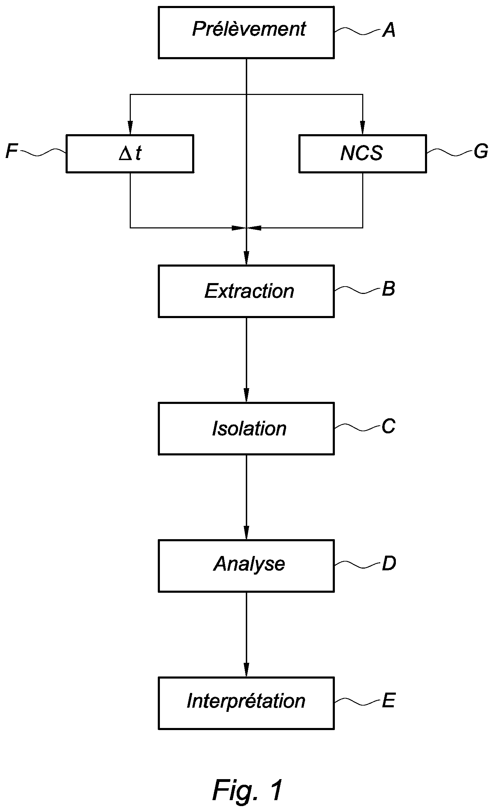

On se réfère d'abord à la figure 1 qui est un organigramme représentant les différentes étapes d'un mode de réalisation du procédé selon l'invention d'évaluation par biodosimétrie de la dose d'irradiation reçue par un individu ayant été soumis à un rayonnement ionisant.

Dans ce mode de réalisation, le procédé comprend des étapes essentielles et des étapes facultatives. Les étapes essentielles sont une étape de prélèvement A de matériels biologiques d'au moins une zone du corps de l'individu, une étape d'extraction B et d'isolation C des protéines contenues dans ces matériels biologiques, et une étape d'analyse D et d'interprétation E des résultats d'analyse pour déterminer la dose d'irradiation reçue dans la ou chaque zone de prélèvement. Les étapes facultatives F et G seront décrites dans le détail dans ce qui suit.

L'étape de prélèvement A comprend le prélèvement, dans au moins une zone du corps de l'individu, de poils et/ou de cheveux et/ou de sang. Les poils et les cheveux comprennent des follicules pileux ou bulbes dont les cellules contiennent des protéines. Le sang comprend des globules rouges et des cellules d'intérêt qui contiennent également des protéines. L'analyse des protéines contenues dans les follicules pileux permet d'évaluer de manière locale la dose d'irradiation reçue par un individu alors que l'analyse des protéines contenues dans les cellules du sang permet d'évaluer de manière globale la dose d'irradiation reçue par l'individu.

Comme expliqué dans ce qui précède, une des conséquences cellulaires majeures des radiations ionisantes est les cassures double brin (CDBs) de l'ADN. La proportion de cassures double brin de l'ADN chez un individu est directement proportionnelle à la dose d'irradiation reçue par cet individu. La mesure de ces cassures permet donc de déterminer la dose d'irradiation reçue par l'individu.

Les cassures double brin de l'ADN induisent une série de réponses cellulaires, dont l'activation de systèmes protéiques et enzymatiques pour signaler et réparer les dommages à l'ADN. Cette réponse fait notamment intervenir les protéines du système ATM, appelé aussi cascade ATM. Certaines protéines du système ATM tel que H2AX, Chk2, NBS1, ATM, BRCA1, 53BP1, P53, KU70, etc. sont phosphorylées alors que d'autres sont déphosphorylées. Une des protéines phosphorylées de manière précoce en réponse aux cassures double brin est l'histone H2AX. Cette histone est phosphorylée en $\gamma$-H2AX.

Dans le cas où un individu a été irradié dans une zone du corps, l'étape d'analyse des cellules prélevées dans cette zone révèlera la présence d'au moins un type protéines phosphorylées du système ATM dont la quantification permettra d'évaluer la dose d'irradiation reçue dans cette zone. Un type de protéines du système ATM s'entend comme H2AX, Chk2, NBS1, ATM, BRCA1, 53BP1, P53 ou KU70. Chaque type de protéines comprend les formes phosphorylée et non phosphorylée de ce type (par exemple H2AX et $\gamma$-H2AX). En variante, l'analyse des cellules prélevées dans chaque zone révèlera la présence d'au moins un type protéines acétylées.

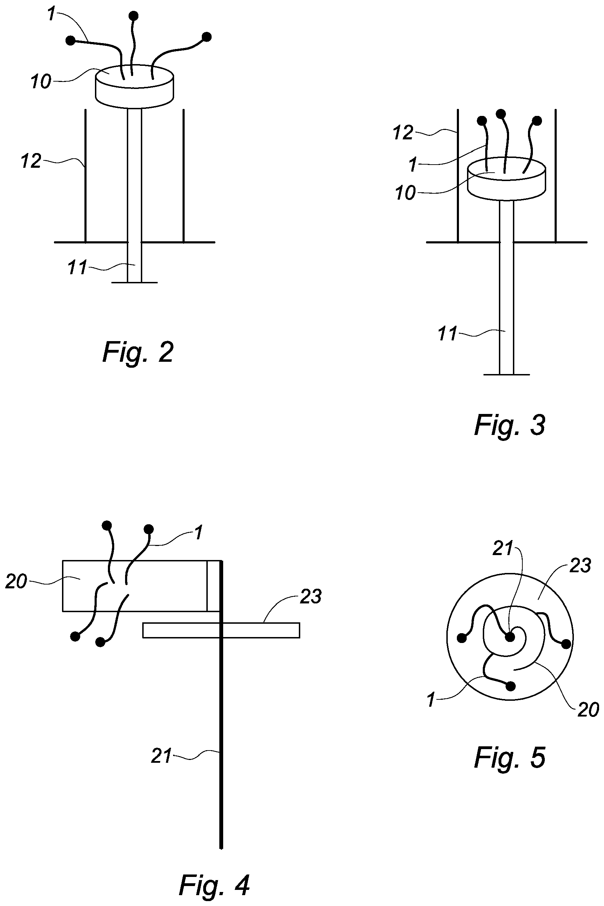

Les figures 2 à 5 représentent deux modes de réalisation d'un dispositif de prélèvement de poils. Dans ces deux modes de réalisation, le prélèvement se fait par l'application d'un patch sur la peau de l'individu au moyen d'un applicateur spécial permettant de replier les follicules pileux après arrachage des poils dans le but de pouvoir les introduire facilement dans un puits de réaction. Le patch peut être composé de cire, de colle, de glue ou de toute autre matière collante permettant de réaliser une épilation.

Dans le mode de réalisation des figures 2 et 3, le patch 10 a une forme de pastille fine à contour sensiblement cylindrique et est fixé à une extrémité d'un piston 11. Ce piston est mobile en translation à l'intérieur d'un tube 12, depuis une position avancée ou le patch 10 est sorti du tube et peut être appliqué sur la peau (figure 2) et une position reculée où le patch est logé dans le tube (figure 3).

Après application du patch 10 (dans sa position avancée) sur la zone de prélèvement à l'aide du piston 11, le retrait du patch 10 entraîne l'arrachage des poils 1 (figure 2). Le déplacement du piston 11 dans le tube 12 (figure 3) jusqu'à sa position reculée, entraîne le repliement des poils 1 qui pénètrent ainsi facilement dans le tube 12.

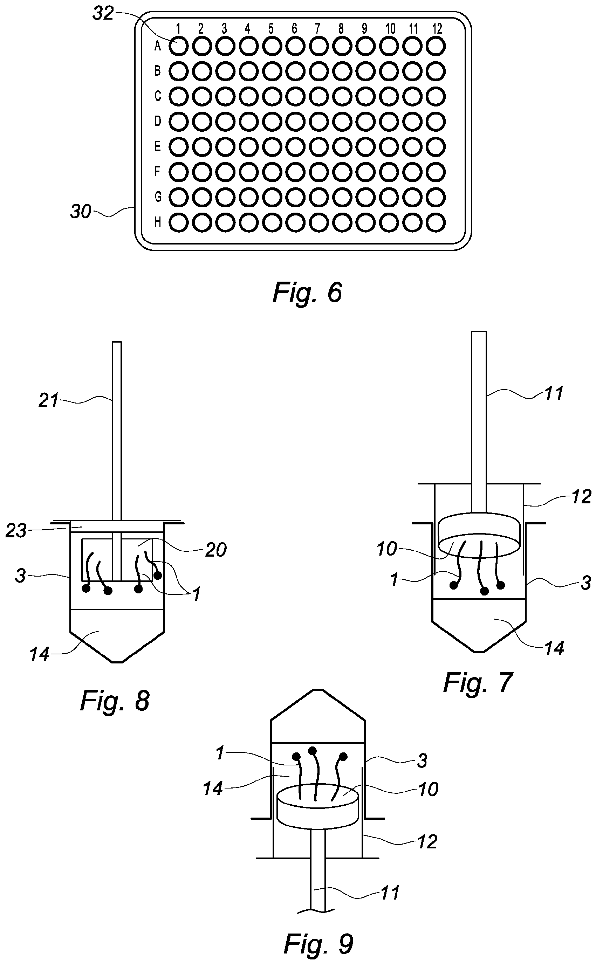

Comme cela est représenté en figure 7, l'extrémité ouverte du tube 12, par laquelle le patch 10 peut sortir, est configurée pour pouvoir être emboîtée dans un puits de réaction 3. Les poils qui sont logés dans le tube 12 sont ainsi facilement introduits dans le puits de réaction 3. Une fois emboîté dans le puits de réaction 3, le dispositif de prélèvement a pour fonction de fermer de manière étanche le puits de réaction.

Dans le mode de réalisation des figures 4 et 5, le patch 20 a une forme de bandelette flexible sensiblement rectangulaire dont une extrémité est fixée à une extrémité d'une tige 21 de façon à ce que la bandelette s'étende sensiblement dans un plan passant par l'axe longitudinal de la tige 21.

La tige 21 porte, à proximité du patch 20, une collerette annulaire externe formant un bouchon 23. Après application du patch 20 sur la peau, son retrait entraîne l'arrachage des poils 1 (figure 4). Le patch 20 est alors enroulé autour la tige 21, c'est-à-dire autour de l'axe longitudinal de la tige. Dans sa position enroulée représentée en figure 5, le patch 20 ne dépasse pas de la périphérie externe du bouchon 23. Le patch peut être maintenu dans cette position à l'aide d'un adhésif (par exemple double face). Le patch 20 peut ainsi être facilement introduit dans un puits de réaction 3, comme représenté en figure 8, le bouchon 23 étant destiné à fermer hermétiquement le puits de réaction 3.

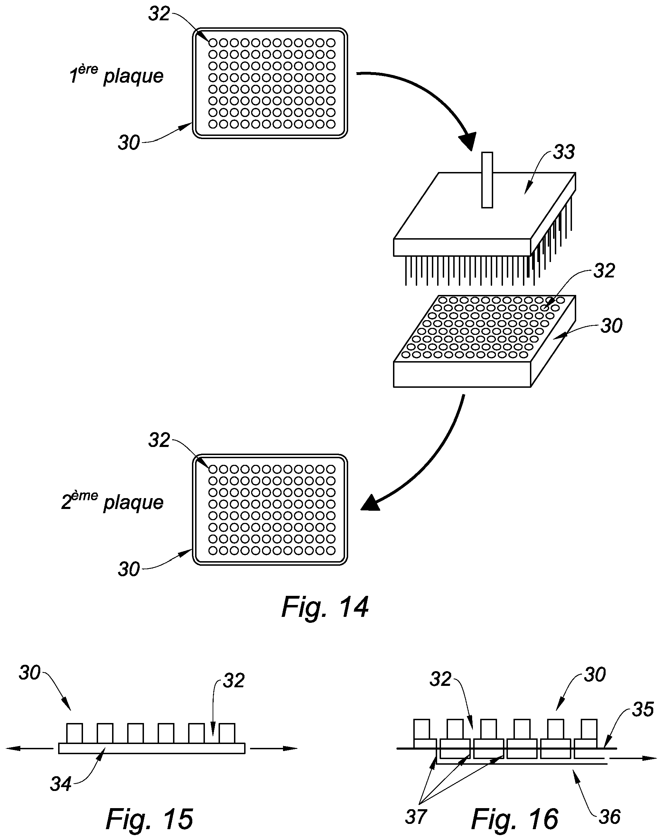

De façon préférentielle, le puits de réaction 3 est le puits 32 d'une plaque multi-puits 30 (figure 6). Chaque puits 32 comprend de préférence les poils d'un prélèvement donné à un instant donné et sur une zone donnée d'un individu donné. L'utilisation d'une plaque multi-puits 30 a notamment pour avantage de pouvoir prélever, ajouter et/ou transférer le contenu des puits 32 en une seule fois à l'aide d'une pipette multicanaux 33, par exemple de manière automatisé (figure 14).

Après introduction du patch 10 dans le puits de réaction, les poils 1 sont mis en contact avec soit une solution 14 non-dénaturante ou de lyse cellulaire. Cette étape peut être réalisée, par exemple, par les sous-étapes successives suivantes : ajout de la solution 14 dans le puits de réaction 3, introduction du patch dans le puits de réaction jusqu'à obturation étanche du puits (figure 7 ou 8), puis retournement du puits de réaction ou de la plaque multi-puits 30 de sorte que les poils soient mis en contact avec la solution 14 (figure 9).

Selon un protocole d'extraction de protéines à partir de poils prélevés, le matériel utilisé comprend un micro-onde, une plaque 96 puits (Eppendorf®) avec couvercle, et une bande de cire (par exemple Nair®). Des bandes de cire de 1cm sur 3 cm sont découpées. La zone de prélèvement est épilée au moyen d'une bande. Une solution de 50 μL de TBS 1X est introduite au fond de trois puits de réaction de la plaque. La bande est collée sur les ouvertures des puits, de façon à ce que les poils soient situés dans les puits et que ces puits soient fermés hermétiquement par la bande. La plaque est fermée avec son couvercle puis est retournée en tapant dessus pour faire tomber la solution sur les poils et la bande. La plaque est mise au micro-onde 15 secondes à 450W. La plaque est retournée en tapant dessus pour faire tomber la solution au fond des puits de réaction. Le couvercle et la bande de cire sont retirés. Le contenu des puits de réaction est transféré dans les puits d'une autre plaque pour une étude par dotblot ou autre.

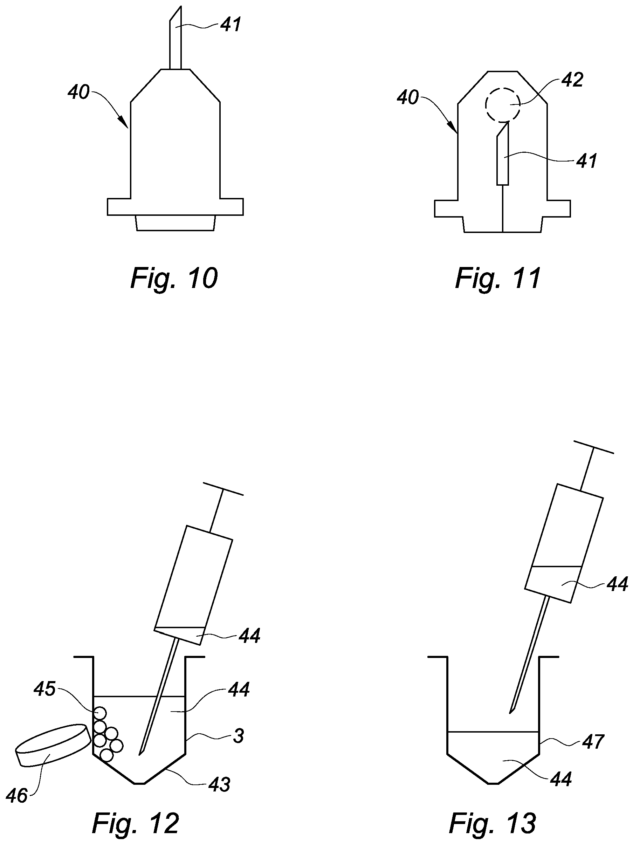

Les figures 10 et 11 représentent un mode de réalisation d'un dispositif de prélèvement de sang, qui comprend ici une lancette 40 comprenant une lame éjectable 41 qui va permettre de piquer et percer la peau, par exemple du doigt, d'un individu. Il peut s'agir d'une lancette jetable similaire à celles destinées aux prélèvements sanguins des diabétiques.

Avant tout prélèvement sanguin, il est nécessaire de désinfecter la zone de prélèvement à l'aide d'une solution antiseptique. De préférence, il s'agira d'alcool. Dans le but de faciliter le prélèvement et de récolter une quantité de sang suffisante pour réaliser le test, il est possible d'appliquer également sur la zone une solution d'anticoagulant, tel que de l'héparine. Ces deux solutions peuvent être appliquées à l'aide de tous moyens d'application, tels qu'une compresse stérile.

Il est également possible de stocker le mélange antiseptique/anticoagulant dans une chambre de stockage 42, qui est logée à l'intérieur de la lancette, sur la trajectoire d'éjection de la lame 41, et qui va être percée lors de l'éjection de la lame 41 de façon à ce que le mélange soit répandu sur la zone de prélèvement simultanément au perçage de la peau (figure 11).

Pour réaliser la totalité des tests de biodosimétrie, 120 à 150 μL de sang peut être collecté par individu dans un puits de réaction 43, tel qu'un tube gradué (figure 12).

Pour éviter toute hémorragie, cette étape de prélèvement peut être suivie d'une étape supplémentaire consistant à appliquer une solution de coagulant, tel que du chlorure de calcium ($CaCl_2$), sur la zone de prélèvement. Cette étape peut être très utile dans le cas d'un prélèvement sur un individu hémophile par exemple. Une compresse contenant une solution de fibronectine peut être appliquée après l'application de la solution de chlorure de calcium

La dosimétrie se fait sur des cellules du sang, à l'exception des globules rouges, les globules rouges sont donc séparés de ces cellules pour faciliter l'analyse. Pour cela, le puits de réaction 43 contient préalablement une suspension de billes magnétiques 45 fonctionnalisées avec des anticorps contre des antigènes de groupe sanguin (tels que anti A, anti B, anti rhésus +, anti CD235, etc.) et éventuellement un anticoagulant pour éviter la coagulation naturelle du sang. Les billes magnétiques fonctionnalisées vont causer la formation d'un caillot de globules rouges tandis que les autres cellules vont rester dans le liquide résiduel 44. Le caillot 45 est formé en 1 à 4 minutes environ. Lors du prélèvement du liquide résiduel 44, le caillot et les billes magnétiques sont ensuite séparés du liquide résiduel à l'aide d'un aimant 46 (figure 12).

50 μl de liquide résiduel peut être introduit dans un second puits de réaction 47 (figure 13) qui est ensuite fermé hermétiquement. Le second puits de réaction peut être un puits 32 d'une plaque multi-puits 30 (figure 6). Chaque puits comprend de préférence le liquide résiduel prélevé chez un individu donné à un instant donné.

Le prélèvement sanguin est réalisé préférentiellement avant le prélèvement des poils. Ainsi, le prélèvement des poils peut être réalisé, par exemple, pendant le temps d'attente de la formation des caillots provoquée par la suspension de billes magnétiques fonctionnalisées avec des anticorps.

Selon un protocole particulier de prélèvement sanguin, le matériel utilisé comprend des billes magnétique anti-IGM de souris (Ademtech), des anticorps (IgM) de souris anti groupe sanguin (A, B, CD235a, etc.), un aimant, et le sang prélevé. 20μL de billes anti-IGM et 15 μL d'anticorps anti-groupe sanguin sont ajoutés dans un tube (par exemple Eppendorf®) de 500μL. Une goutte de sang (entre 20 et 50 μL) est ajoutée dans le tube. Après une attente de 15 secondes environ (présence d'un vortex éventuel), le tube est placé contre l'aimant pendant environ une minute. Une fois que les billes ont emportées le maximum d'hématie, le plasma sanguin comportant les leucocytes et thrombocytes peut être prélevé et étudié.

L'étape suivant le prélèvement A est l'étape d'extraction B des protéines des cellules contenues dans les matériels biologiques prélevés. Cette étape consiste soit (i) à mettre en contact les cellules avec une solution de lyse cellulaire, soit (ii) à mettre en contact les cellules avec une solution non dénaturante puis à soumettre les cellules à une lyse mécanique ou par l'intermédiaire d'ondes.

Dans le premier cas (i), dans un mode préférentiel de réalisation, une solution de lyse cellulaire 14 est présente dans chaque puits de réaction 3. Dans le cas du prélèvement de poils, ceux-ci peuvent être mis en contact avec cette solution, par exemple par retournement du puits de réaction, comme représenté en figure 9. Dans le cas où le puits de réaction appartient à une plaque 30, cette plaque peut être retournée pour retourner simultanément l'ensemble des puits de réaction.

Dans le cas du prélèvement de sang, le liquide résiduel 44 contenant les cellules sanguines d'intérêt peut être mis en contact avec la solution de lyse cellulaire lors du transfert du liquide résiduel dans un puits de réaction 47, comme représenté aux figures 12 et 13.

La mise en contact des cellules du sang ou des follicules pileux avec la solution de lyse cellulaire provoque la destruction des cellules et la libération des protéines contenues dans ces cellules. L'extraction protéique peut durer entre 15 secondes à 3 minutes environ.

Dans le second cas (ii), les puits de réaction peuvent contenir une solution non-dénaturante telle que du TBS, du TBE, etc., de préférence exempte de phosphore pour ne pas perturber les analyses par LIBS. La lyse peut être provoquée de plusieurs façons différentes et de préférence par des micro-ondes, de façon à ne pas modifier l'état de phosphorylation des protéines. La plaque multi-puits peut être placée, après retournement, entre 1 à 60 secondes sous une source micro-ondes de manière à faire éclater les cellules et libérer les protéines qu'elles contiennent.

Après extraction B des protéines 51, celles-ci sont capturées et isolées C. Elles peuvent être capturées :

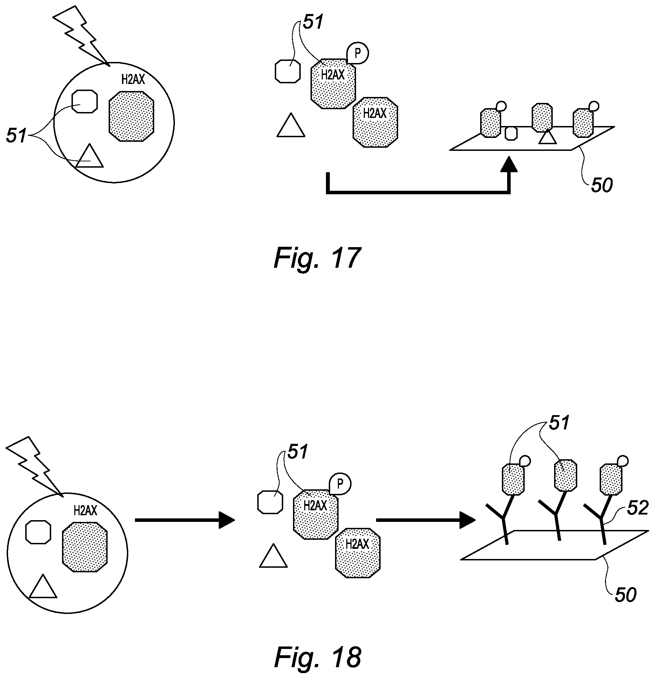

- soit directement sur les fonds 50 des puits de réaction, de façon à former des systèmes non-structurés (figure 17),
- soit par l'intermédiaire d'anticorps 52, de façon à former des systèmes structurés après transfert dans une plaque à puits où le fond des puits comprend un système d'anticorps structuré où par exemple chaque type d'anticorps est imprimé dans la structure d'un motif, par exemple à l'aide d'une imprimante à jet d'encre (figure 18).

Dans chaque système non-structuré (figure 17), tous les types de protéines sont absorbées sur le fond 50 d'un puits de réaction, sous leurs formes phosphorylée et non-phosphorylée. Dans l'exemple représenté en figure 17, les éléments de forme sensiblement octogonale représentent un type de protéines, les histones H2AX. Lorsque ces protéines sont phosphorylées (y-H2AX), elles portent un élément marqué P. Dans le fond du puits de réaction de la figure 16, trois protéines du type H2AX sont présentes dont deux phosphorylées (y-H2AX) et une non-phosphorylée (H2AX).

Dans chaque système structuré (figure 18), le fond 50 de chaque puits est structuré avec des anticorps 52 greffés sur ce fond. Ces anticorps 52 sont dirigés contre un unique type de protéines. Dans l'exemple représenté, les anticorps sont destinés à se lier spécifiquement aux protéines H2AX, dans leurs formes phosphorylée et non-phosphorylée. Dans le fond du puits de réaction de la figure 18, trois protéines du type H2AX sont présentes dont deux phosphorylées (y-H2AX) et une non-phosphorylée (H2AX). Les autres types de protéines ne sont pas retenus sur le fond du puits et peuvent être éliminées.

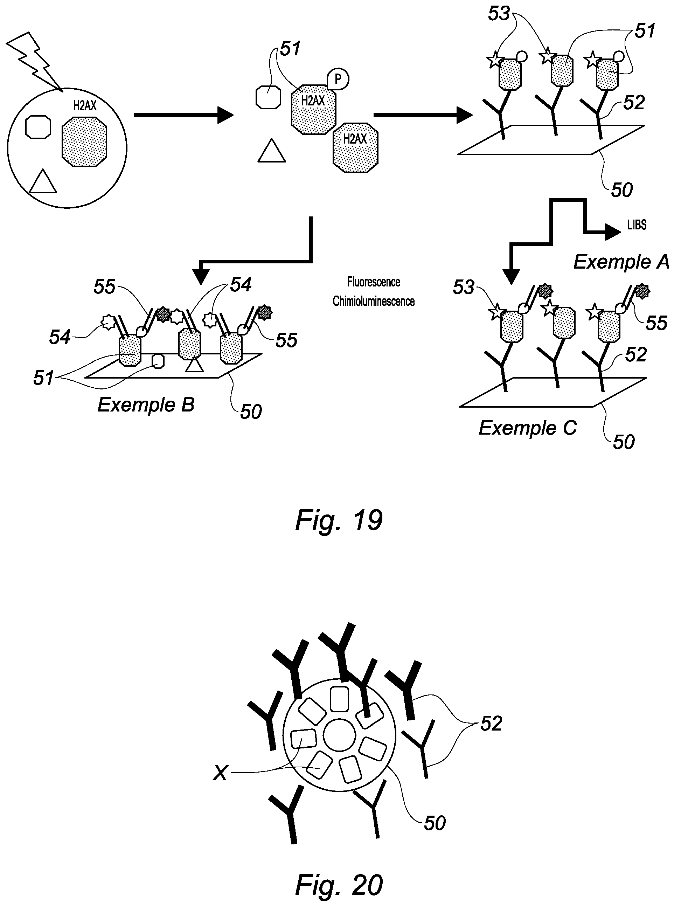

Des anticorps différents destinés à se lier spécifiquement à différents types de protéines peuvent être greffés au fond de chaque puits de réaction, comme cela est schématiquement représenté en figure 20. Le fond 50 du puits comprend plusieurs zones X distinctes de fixation d'anticorps 52, les anticorps d'une zone étant différents des anticorps des autres zones et étant destinés à se lier à un type de protéines différent des types de protéines destinés à se fixer aux anticorps des autres zones. Les zones X peuvent comprendre par exemple des anticorps correspondant respectivement à H2AX, Chk2, NBS1, ATM, BRCA1, 53BP1, P53 et KU70, dans leurs formes phosphorylée et non-phosphorylée.

A l'issue de l'étape d'isolation, les protéines à analyser sont donc retenues sur les fonds des puits de réaction, selon un mode de réalisation particulier du procédé selon l'invention. De façon préférentielle, la plaque multi-puits 30 comprendra un fond amovible 34 qui permet de faciliter la lecture des résultats lors de l'analyse (figure 15).

Le fond peut également comprendre ou être formé d'une membrane filtrante 35 au travers de laquelle les solutions contenues dans les puits 32, et éventuellement certaines molécules de taille donnée peuvent être aspirées (figure 16). L'aspiration peut être réalisée par une plaque 36 de support destinée à recevoir la plaque multi-puits 30 et comportant des capillaires d'aspiration 37 qui comportent chacun une première extrémité débouchant en regard d'un puits 32 et une seconde extrémité reliée à une source d'aspiration (figure 16). Un tel système permet de vider très rapidement tous les puits de réaction 32 pour accélérer et faciliter par exemple la fixation des protéines sur le fond des puits.

Le fond peut également comprendre ou être formé d'une membrane capable d'absorber les protéines. Il peut s'agir d'une membrane en PVDF (pour *polyvinylidene difluoride* en anglais), en nitrocellulose ou en époxy.

Après isolation C des protéines 51, différentes solutions existent pour leur analyse D.

Dans un mode préféré de réalisation du procédé, l'analyse peut être réalisée par la mesure de l'intensité (IP) d'un marqueur propre à la forme phosphorylée d'un type de protéines considéré, ou par le ratio de cette intensité (IP) sur celle (I) d'un marqueur des formes phosphorylée et non-phosphorylée de ce type de protéines (ce ratio IP/I correspondant au taux de phosphorylation de ce type de protéines). Par comparaison à des courbes d'étalonnage préétablies, il est possible de déterminer la dose d'irradiation reçue par un individu.

La figure 19 représente de manière schématique trois exemples A, B et C de marquage des protéines au fond des puits de réaction.

Selon l'exemple A, toutes les protéines (formes phosphorylée et non-phosphorylée) du type de protéines retenu sur le fond du puits de réaction, comme expliqué en référence à la figure 18, sont marquées au bore 53 en vue d'une analyse LIBS.

Selon l'exemple B, comme expliqué dans ce qui précède en référence à la figure 17, toutes les protéines 51 contenues dans un puits de réaction se sont déposées sur le fond de ce puits. Les formes phosphorylée et non phosphorylée d'un type de protéines sont marquées au moyen d'un premier marqueur 54, et la forme phosphorylée de ce type de protéines est marquée au moyen d'un second marqueur 55.

L'exemple C est similaire à l'exemple A mais comprend en outre des marqueurs 56 propres à la forme phosphorylée du type de protéines considéré.

Dans les exemples B et C, le marquage des protéines peut être réalisé au moyen d'anticorps, ces anticorps pouvant être des molécules chimériques telles que décrites dans la demande antérieure PCT/FR2011/050812 de la demanderesse.

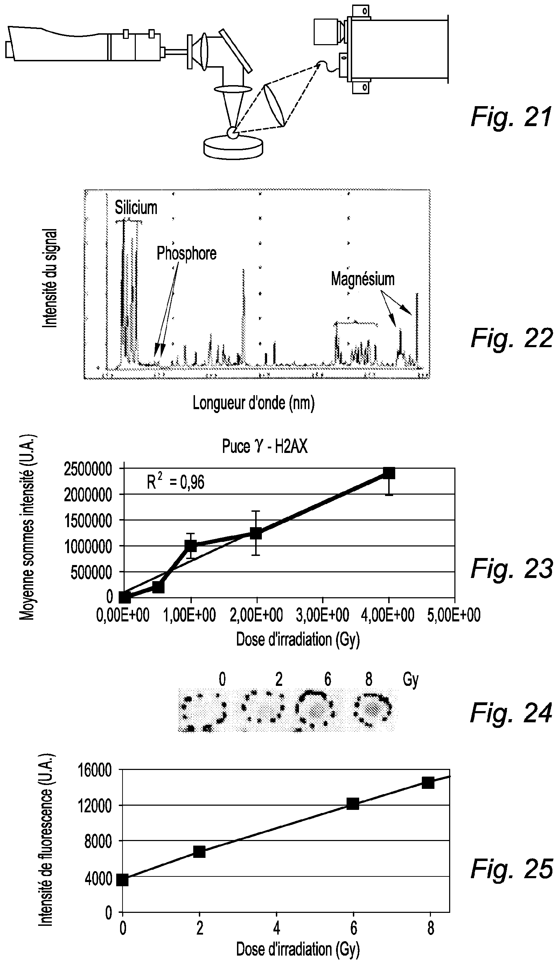

Dans l'exemple A, le fond 50 du puits de réaction ou de la plaque multi-puits peut être analysé par une méthode pouvant mesurer directement le taux de phosphore des protéines phosphorylées. Il peut s'agir d'une méthode d'analyse avec un laser de type LIBS (figure 21). Cette méthode permet d'obtenir un spectre (figure 22) permettant de déterminer l'intensité (IP) des signaux correspondant au phosphore, qui est proportionnelle à la quantité de protéines phosphorylées, ainsi que l'intensité (I) des signaux correspondant au bore (marqueur 53), qui est proportionnelle à la quantité de protéines phosphorylées et non phosphorylées. Par comparaison à des courbes d'étalonnage, telles que celle représentée en figure 23, il est possible de déterminer la dose d'irradiation reçue par un individu.

La courbe d'étalonnage de la figure 23 représente l'évolution de l'intensité IP en fonction de la dose d'irradiation reçue par un individu. Cette courbe a été tracée à partir de quatre points correspondant respectivement à quatre tests effectués sur quatre individus différents. Des poils de chaque individu ont été prélevés et soumis à des rayonnements ionisants à une dose prédéterminée. Leurs protéines ont été extraites et isolées, comme décrit dans ce qui précède, puis analysées par la méthode LIBS. Les résultats obtenus ont permis d'établir la courbe de la figure 23, qui permet de déterminer la dose d'irradiation reçue par un individu à partir de l'intensité IP. Une courbe d'étalonnage du type de celle de la figure 23 est propre à un type de protéines donné et à un paramètre ou une grandeur étudié donné. Dans l'exemple précité, le paramètre observé est IP. En variante, le paramètre étudié est le ratio IP/I. La courbe d'étalonnage est alors du type IP/I = f(D), D étant la dose d'irradiation.

On a vérifié que les résultats obtenus par l'étape d'analyse LIBS sont proches de ceux obtenus avec la méthode de tri cellulaire dite FACS de la technique antérieure, ce qui permet de valider les résultats.

Dans les exemples B et C précités, les protéines marquées peuvent être analysées par fluorescence, chimioluminescence ou colorimétrie, les marqueurs 53, 54, 55 étant des marqueurs fluorescents, colorimétriques ou chimioluminescents.

La Figure 24 représente l'intensité de fluorescence de protéines $\gamma$-H2AX, capturées directement sur le fond de quatre puits de réaction pourvus d'une membrane PVDF, ces protéines ayant été soumises à des doses d'irradiation différentes (0, 2, 6 et 8 Gy). On peut observer l'augmentation de l'intensité IP de fluorescence en fonction de l'augmentation de la dose d'irradiation et une courbe d'étalonnage peut être établie (IP = f(D) - figure 25). Cette courbe permet de déterminer la dose d'irradiation reçue par un individu en fonction de l'intensité de fluorescence mesurée, propre à la forme phosphorylée d'un type de protéines par exemple. Comme indiqué dans ce qui précède, la courbe d'étalonnage peut être du type IP/I = f(D).

Dans une variante de réalisation du procédé selon l'invention, après extraction, séparation et marquage des protéines, comme décrit dans ce qui précède, les protéines marquées sont transférées dans les puits d'une plaque multi-puits, de sorte que chaque puits comprenne un mélange de particules sur lesquelles sont fixés des anticorps contre des marqueurs donnés, par exemple des marqueurs de la forme non phosphorylée d'un type de protéines.

Ces particules ont de préférence des tailles micrométriques, par exemple comprises entre 10nm et 100mm. Il pourra s'agir d'un mélange de particules tel que chaque particule comprend un seul type d'anticorps.

Dans cette configuration, à un type d'anticorps, une taille de particules unique pourra être attribuée de manière à pouvoir distinguer les particules par leur taille et à pouvoir en déduire les anticorps qui y sont fixés. Dans d'autres cas, chaque particule peut comprendre un mélange d'anticorps.

Une fois les marqueurs capturés par les anticorps des particules, différentes analyses peuvent être réalisées :

- les particules peuvent être piégées par filtration sur la membrane constituant le fond de la plaque multi-puits, comme expliqué dans ce qui précède, la membrane constituant un filtre dont les ports ont une taille inférieure à la taille des particules,
- un cocktail d'anticorps comprenant des anticorps dirigés contre d'autres épitopes que les épitopes reconnus par les anticorps sont fixés sur les particules, et tel que chaque type d'anticorps comporte un marqueur fluorescent donné, peut être présent ou ajouté avec les particules. Chaque marqueur fixé sur une particule sera alors marqué par un ou deux anticorps différents supplémentaires ; de manière préférentielle, par un anticorps contre la forme phosphorylée du marqueur recherché.

Après filtration, les particules retenues sur le fond de la plaque multi-puits comporte les marqueurs piégés par les particules, les marqueurs étant marqués d'une part de manière ubiquitaire ou par un anticorps, et d'autre part par les marqueurs phosphorylés, et éventuellement en plus marqués par un anticorps contre la forme phosphorylée. Le fond de la plaque est ensuite analysé par l'une des méthodes précitées.

Une autre alternative est l'analyse des particules de chaque puits de la plaque par FACS.

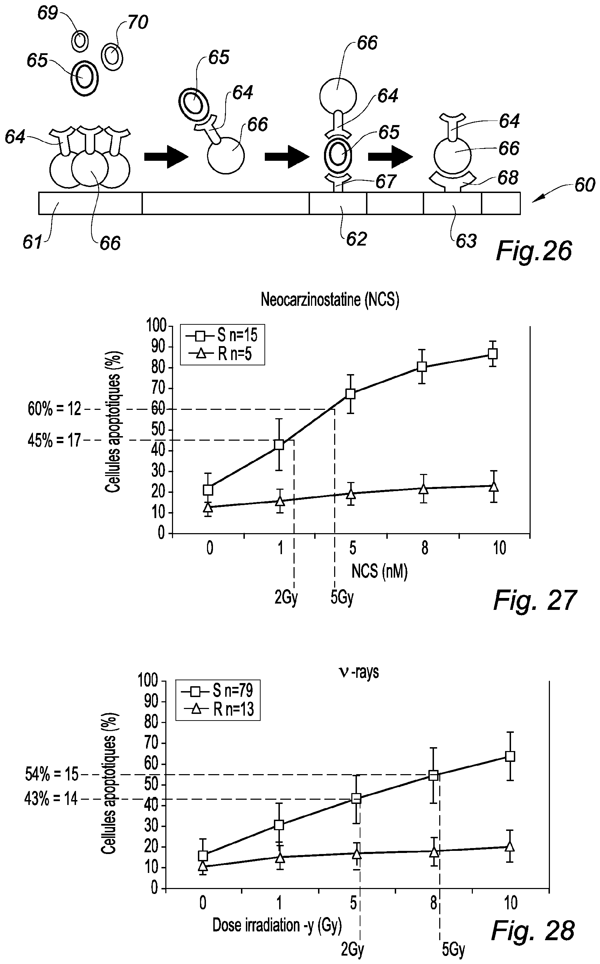

Dans une autre variante de réalisation du procédé selon l'invention représentée à la figure 26, après extraction, les protéines sont transférées dans les puits d'une plaque multi-puits, de sorte que le fond de chaque puits soit recouvert d'une bandelette 60 en papier Whatman®, cette bandelette ayant une forme allongée et définissant à une extrémité une zone 61 de dépôt des protéines à analyser, et à l'extrémité opposée des zones d'analyse 62, 63. Les protéines sont destinées à migrer de la zone de dépôt à la zone d'analyse par chromatographie.

La zone de dépôt 61 comprend des anticorps 64 contre la forme phosphorylée 65 d'un type de protéines (ici, $\gamma$-H2AX), ces anticorps 64 étant fixés à des particules 66, telles que des billes d'or.

La première zone d'analyse 62 comprend des anticorps fixes 67 contre les formes phosphorylée et non phosphorylée du type de protéines (ici, H2AX). La seconde zone d'analyse 63 comprend des anticorps fixes 68 destinés à capturer les particules 66 dont les anticorps 64 ne sont pas liés à des protéines (car les particules 66 dont les anticorps 64 sont liés à des protéines 65 sont retenues dans la zone 62).

Une solution contenant les protéines à analyser est déposée dans la zone 61 de la bandelette 60, cette solution comprenant le type de protéines étudié dans ses formes phosphorylée 65 ($\gamma$-H2AX) et non phosphorylée 69 (H2AX). Cette solution peut être obtenue par extraction des protéines de sang ou de poils, comme décrit dans ce qui précède. Une quantité prédéterminée de protéines marquées 70 du type précité, dans leur forme non phosphorylée, est ajoutée à la solution avant dépôt sur la bandelette.

Lorsque cette quantité connue de protéines marquées 70 est déposée seule et analysée avec la bandelette 60, les résultats obtenus, par exemple par colorimétrie, fournissent un signal dans la zone 62 dont l'intensité i* est maximale (100%) car toutes les protéines marquées sont retenues dans cette zone.

Lors d'un test, l'intensité i du signal obtenu dans la zone 62 sera d'autant plus diluée que le nombre de protéines du type précité (dans ses formes phosphorylée et non phosphorylée) sera retenue dans cette zone. Les valeurs de i* et i permettent de déterminer le paramètre I, c'est-à-dire la quantité de protéines du type précité, extraite du matériel biologique prélevé sur l'individu, par la formule : I = (i* - i). Le paramètre IP est obtenu par l'intensité du signal généré par les billes 65 dans la zone 62. Les paramètres i/i* (c'est-à-dire, léchantillon/Iref) ou i*/I (Iref/I) peuvent également être étudiés.

Comme précédemment, par comparaison à une courbe d'étalonnage, il est possible de déterminer la dose d'irradiation reçue par un individu.

La modification du taux de phosphorylation des protéines impliquées varie notamment selon la vitesse de réparation des cassures double brin de l'ADN. Cette vitesse de réparation est différente d'un individu à l'autre (variations interindividuelles). Il est donc avantageux de mesurer cette vitesse afin de corriger la mesure et obtenir des résultats beaucoup plus précis (étape F en figure 1).

La détermination de cette vitesse peut être effectuée par le biais de deux prélèvements sanguins ou pileux dans une même zone. Ces prélèvements sont effectués à un intervalle de temps $\Delta t$ prédéterminé ou sont réalisés quasi-simultanément et au moins l'un des prélèvements est mis en culture pendant une durée déterminée $\Delta t$. La durée $\Delta t$ peut être comprise entre 10 minutes et 6 heures.

Chaque prélèvement est traité et analysé comme décrit précédemment. Les mesures d'intensité IP ou de ratio IP/I obtenues peuvent être accompagnées de la dérivée ou de l'accroissement fini de cette valeur. On a ainsi accès au couple de paramètres (IP/I, $\partial$(IP/I)/ $\partial$t) ou (IP, $\partial$IP/$\partial$t). Les paramètres $\partial$(IP/I)/$\partial$t et $\partial$IP/$\partial$t peuvent ainsi être étudiés et les courbes d'étalonnage peuvent être du type $\partial$IP/$\partial$t = f(D) ou $\partial$(IP/I)/$\partial$t= f(D).

Il est également possible de corriger les erreurs dues à la variation interindividuelle en utilisant une substance chimio radiomimétique. Il s'agit d'un mime chimique de l'irradiation, i.e., une substance qui, lorsqu'elle est ajoutée à du matériel biologique, provoque des cassures double brin de l'ADN (étape G en figure 1).

La substance utilisée est par exemple la néocarzinostatine (NCS). Le graphe de la figure 27 représente l'évolution du ratio de cellules apoptotiques d'un mélange en fonction de la concentration en NCS dans ce mélange, pour deux lignées différentes de cellules, respectivement sensible S et résistante R. Le graphe de la figure 28 représente l'évolution du ratio de cellules apoptiques d'un mélange en fonction de la dose d'irradiation reçue par ce mélange, pour les deux lignées précitées. Ces graphes ont permis de déterminer notamment qu'une concentration de 1 nM de néocarzinostatine est équivalent, c'est-à-dire produit les mêmes effets, qu'une dose d'irradiation de 1,2 Gy.

Plusieurs prélèvements pileux sont effectués sur une même zone d'un individu, de même que plusieurs prélèvements sanguins. Deux des prélèvements sont soumis à l'action du NCS à dose connue, par exemple équivalente à 1Gy, dans des puits de réaction d'une plaque. Chaque prélèvement peut être traité et analysé, comme décrit précédemment. Un des deux prélèvements est analysé immédiatement en même temps qu'un prélèvement n'ayant pas reçu de traitement, le second est analysé après une durée déterminée $\Delta t$, par exemple 1 heure.

Les mesures d'intensité IP ou de ratio IP/I obtenues peuvent être accompagnées, en plus des dérivées de ces valeurs, à des paramètres tenant compte des variations interindividuelles des individus. Les mesures IP/I obtenues sont alors modulées par $\partial$(IP/I) traité / $\partial$t et par (IP/I)/ (IPtraité/Itraité).

Des courbes et/ou des surfaces d'étalonnage peuvent ainsi être réalisées par combinaison linéaire ou non linéaire de tout ou partie de ces grandeurs et de leur ratio (par exemple l'abscisse X est le temps t, Y est un paramètre étudié tel que IP, et Z est la dose d'irradiation). L'ensemble des valeurs et/ou combinaison linéaire ou non linéaire de grandeurs provenant des mesures de phosphorylation pouvant être des paramètres étudiés.

D'une manière générale, dans ce qui suit, la valeur retenue pour mesurer le taux de phosphorylation sera appelée l'observable.

Dans certains modes de réalisation, afin d'affiner la corrélation de la mesure de l'observable à la dose reçue, le procédé prend en compte la formule sanguine des individus. La formule sanguine des individus est donc réalisée systématiquement au moment du prélèvement sanguin tel que pour un individu i, les fractions sanguines des différents types cellulaires j composant son sang soient définies par $X_j$. De nombreux automates portables permettent de déterminer en quelques minutes la formule sanguine d'un individu, l'objectif étant de déterminer quelle fraction de la formule sanguine il est nécessaire de considérer pour corréler au mieux les mesures de phosphorylation à la dose reçue.

Ainsi un procédé selon l'invention comprend une étape de recherche d'une combinaison linéaire de la formule sanguine en fonction de l'observable et maximisant la corrélation à la dose reçue.

Les courbes ou les surfaces étalons pour réaliser cette dosimétrie seront réalisées à partir d'une population témoin.

De manière préférentielle, la population témoin comprendra P personnes, P étant égal ou supérieur à N qui représente le nombre de fractions énumérées dans la formule sanguine considérée.

Les intensités IP ou les ratios IP/I, ou plus généralement l'observable, sont déterminées sur les cellules de sang des prélèvements sanguins des individus, comme expliqué dans ce qui précède. L'observable est mesuré pour différentes doses variant par exemple de 0 à 8 Gray, par pas de 0,1 Gray, et pour différents temps après l'exposition variant par exemple de 0 à 12 h, par pas de 30 mn.

Pour chaque temps t et chaque dose $D_d$ reçue par un individu i, la formule sanguine est réalisée et l'équation :

$$\left( \Sigma_{j=1}^{N} A_{dtij} X_{dtij} \right) (\text{observable}_{dti}) + f_{dti} = D_d$$

est établie où $X_{dtij}$ représente la fraction cellulaire j pour un individu i, $A_{dtij}$ le coefficient de pondération de la fraction $X_{dtij}$, permettant de relier l'observable$_{dtij}$ à la dose $D_d$ ; N représente le nombre de fractions considérées dans la formule sanguine, et $f_{dti}$ le facteur correctif de la corrélation.

Pour estimer les facteurs A à prendre en compte pour chaque dose et à chaque temps, toutes les combinaisons C de N personnes dans P sont considérées.

Pour chaque combinaison C de N personnes et pour chaque temps t et chaque dose $D_d$, le système $[(\Sigma_{j=i}^{N} A_{dtij} X_{dtij}) (\text{observable}_{dti}) + f_{dti} = D_d]$ de N équations à N inconnues est résolu.

Pour chaque système d'équations d'une combinaison C, N jeux de N facteurs $A_{dtj}$ et N facteurs correctifs $f_{dti}$ sont obtenus. Pour chaque temps et chaque dose, et pour chaque fraction J, une valeur moyenne de facteur de pondération $A_{dtj}$ est alors calculée à partir de tous les $A_{dtij}$ de toutes les combinaisons. Pour chaque temps et chaque dose le facteur de correction $f_{dt}$ est calculé à partir de tous les $f_{dti}$ obtenus pour toutes les combinatoires.

Pour toutes les doses d'un même temps, il est alors recherché une combinaison des $A_{dtj}$ où J est compris entre 1 et N, telle que la corrélation : $A_{dtj}$(observable $_{dt}$) versus Dose$_d$ soit la meilleure.

En résumé il est recherché pour chaque temps une combinaison linéaire des $A_{dtj}$ permettant de corréler au mieux la relation dose/effet observée.

Dans un exemple préféré de réalisation, la protéine Ku70 est utilisée comme marqueur pour déterminer la dose d'irradiation reçue par un individu.

En effet, le taux de phosphorylation de la protéine Ku70 est proportionnel à la dose reçue. Plus particulièrement, la quantification de la phosphorylation au niveau de la sérine 27 de la protéine Ku70 permet d'estimer la dose d'irradiation reçue par un individu.

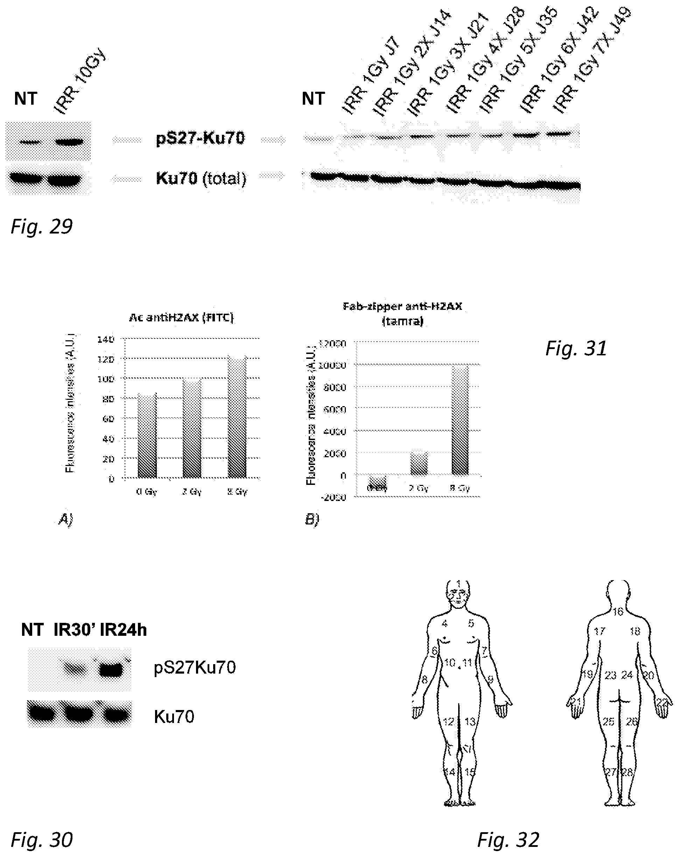

La figure 29 montre que phospho-Ser27-Ku70 est inductible dans les lignées cellulaires (lymphoblastes T) exposées aux rayonnements gamma (irradiation ionisante) à fortes doses uniques (10Gy) ainsi que dans les cellules exposées aux doses fractionnées à raison de 1Gy/semaine pendant 7 semaines (dose totale cumulée 7Gy). L'observation de la variation de K70 permet de quantifier une irradiation chronique

La figure 30 montre que le taux de phosphorylation de la protéine Ku70 varie au cours du temps. Le taux de phosphorylation, 24h après l'irradiation, est nettement supérieur à celui 30 minutes après l'irradiation (à 10Gy).

Dans certains modes de réalisation, la quantification de la phosphorylation sera réalisée grâce à un anticorps dirigé contre un épitope comprenant la sérine 27. Plus particulièrement, l'anticorps sera dirigé contre tout ou partie de la séquence peptidique ENLEA(p)SGDYK. (SEQ ID1) (il s'agit ici de la nomenclature classique des acides aminés, la lettre p représentant toutefois un phosphate).

Dans certains modes de réalisation, la mesure utilisée pour quantifier la dose d'irradiation d'un individu est le rapport du taux de Ku70 phosphorylée (Ku70 p) par rapport à la Ku70 totale (comme présenté aux figures 29 et 30).

La quantité de Ku70 totale reste constante dans les cellules, le ratio Ku70 p / Ku70 totale permet de déterminer la dose d'irradiation reçue. Dans certains modes de réalisation, le dosage de Ku70 totale peut se faire grâce à un anticorps dirigé contre les parties de Ku70 conservées et non modifiées, notamment non modifiées par la phosphorylation qui, de préférence, est éloignée du site de phosphorylation, par exemple à plus de dix acides aminés.

Le dosage de Ku70 totale permet également de normaliser les autres marqueurs utilisés pour doser l'irradiation. Notamment le ratio γH2AX/ Ku70 totale, permet d'évaluer la dose reçue par un individu même six heures après l'irradiation. En effet, il est montré sur FACS que le niveau de phosphorylation de la protéine H2AX varie au cours du temps en raison de la réparation de l'ADN, et que cette variation diffère selon les individus. Le ratio γ H2AX/ Ku70 permet de classer les doses reçues par différents individus en au moins 3 catégories d'irradiation par exemple [0-0.5 Gy] [0.5-3Gry] [>3Gry], jusqu'à six heures après l'irradiation.

Dans certains modes de réalisation :

a. les protéines de l'extrait cellulaire seront capturées sur un support membrane, verre, Kapton® ou tout autre matériau, au travers d'une méthode de dépôt, de filtration, de cytospine. Dans d'autres modes de réalisation, les protéines sont fixées sur des particules et plus particulièrement des particules de diamant, de polystyrène ou de tout autre matériau. La fixation des protéines sur le support peut être covalente, dative (Van der Waals), ou simplement absorbée,

b. les protéines fixées sur la membrane ou les particules sont mises en contact avec les anticorps dirigés contre les cibles tel que phospho Ku70, γ H2AX, et également contre au moins une cible utilisée pour la normalisation telle que Ku70, H2AX ou toute autre protéine s'exprimant de manière constante dans les cellules. Un anticorps dirigé contre une cible aura un marquage fluorescent ou colorimétrique spécifique,

c. après élimination des anticorps marqués non complexés, la lecture de la fluorescence où l'intensité de chaque couleur correspondant à un anticorps donné permet de déterminer l'abondance de la cible et donc le niveau de phosphorylation, notamment en normalisant la mesure par l'intensité de fluorescence d'au moins un anticorps de normalisation tel que I AC cible /I AC normalisation permet de déterminer la proportion de protéines phosphorylées et donc la dose reçue. Dans certains modes de réalisation avec l'utilisation de nanoparticules, la lecture de la fluorescence peut être réalisée par FACS.

Dans certains modes de réalisation :

a. les anticorps sont fixés sur un support tel que chaque anticorps soit déposé en une quantité connue à une coordonnée x,y du support de manière à former un spot ou une unité de complexation. Par exemple, des spots d'anticorps dirigés contre Ku70, phospho Ku70, H2AX, γH2AX, ou tout autre anticorps contre des protéines impliquées dans la voie canonique ATM. Le support comprendra un anticorps dirigé contre une protéine présente dans l'échantillon dont la quantité ne varie pas dans ce dernier,

b. les anticorps organisés en spot sont alors complexés avec un extrait protéique dans lequel les protéines sont marquées par des marqueurs de fluorescence de colorimétrie ou toute chimioluminescence ou tout autre marqueur,

c. élimination des protéines non complexées, lavage et rinçage de la lame, et

d. lecture dans un scanner de fluorescence ou colorimétrique.

Dans certains modes de réalisation, les anticorps et l'échantillon sont complexés directement, après élimination des protéines non complexées et rinçage. L'ensemble complexe échantillon est marqué par des marqueurs fluorescents, colorimétriques, chimioluminescents ou tout autre marqueur capable de se fixer sur les protéines, par exemple sur la fonction amine ou carboxylique, peptidique des protéines. Après élimination des marqueurs n'ayant pas réagi, le complexe anticorps/protéine est scanné et dosé. La quantité de fluorescence indique la proportion de protéines marquées et la concentration d'anticorps. Un spot d'anticorps marqués non complexés permet de déterminer l'intensité de base pour la normalisation. Dans ce mode de réalisation, la saturation du support comportant les anticorps est réalisée par une substance de blocage ne fixant pas le marquage.

Dans certains modes de réalisation, les anticorps sont fixés sur des nano ou micro particules par exemple de polystyrène ou de diamant, éventuellement magnétiques tel qu'un anticorps donné soit fixé sur une particule identifiable de manière univoque, par un marquage fluorescent, par une taille unique, de sorte qu'une particule donnée capture une cible donnée. Les cibles marquées avec un fluorochrome différant du marquage des particules sont mises en contact avec des particules fonctionnalisées par les anticorps, après isolement et lavage des particules, les particules sont analysées en FACS (de préférence un FACS à microcapillaire portable). La mesure de la fluorescence spécifique à une particule ou la mesure de la diffraction qualifiant la taille de la particule permet d'identifier la particule. La mesure de la fluorescence spécifique à l'échantillon sur chaque particule permet de mesurer la quantité de cible capturée par les anticorps liés à la particule et permet de déduire l'abondance des cibles recherchées.

Dans certains modes de réalisation, pour chaque cible est utilisé un anticorps contre l'épitope phosphorylé par exemple ENLEA(p)SGDYK (SEQ ID1) phospho Ku70 et un autre anticorps dirigé contre l'épitope non phosphorylé ENLEASGDYK (SEQ ID2) tel que les protéines phosphorylées soient capturées uniquement par l'anticorps anti forme phosphorylée et que la forme non phosphorylée soit capturée uniquement par l'anticorps contre la forme non phosphorylée. Par ce procédé, il est alors possible de déterminer la proportion de forme phosphorylée comme I anti phospho Ku70/ (I anti phospho Ku70 + I anti Ku70). Le même couple est également développé pour H2AX, $\gamma$ H2AX et tous les autres types de protéines phosphorylées de la cascade ATM.

Dans certains modes de réalisation, les anticorps fixés sur une particule ou un support sont complexés avec un mélange calibré (même concentration en protéines) constitué d'une part de l'échantillon à analyser marqué avec un marqueur fluorescent, colorimétrique, chimioluminescent, etc. : mélange A, et d'autre part d'une référence calibrée pour les cibles recherchées et marquées avec un autre marqueur fluorescent, colorimétrique, chimioluminescent, etc. : mélange B. Les deux marqueurs A et B émettent des longueurs d'onde différentes ne se recouvrant pas. Après complexation, les cibles marquées différemment se concurrencent pour se lier avec l'anticorps complémentaire. Le rapport IA/IB de chaque cible permet de quantifier la cible A, la concentration de la cible référence B étant connue.

Dans certains modes de réalisation, des références proviennent d'extraits de lymphocytes et/ou de cellules folliculaires de poil et ou kératinocyte et/ou cellule immortalisée irradiées à différentes doses. Chaque point de référence est irradié à une dose précise, l'ensemble des points de référence forment une gamme. Chaque point de la gamme est complexé et analysé dans les mêmes conditions que les autres extraits.

Dans certains modes de réalisation, la complexification anticorps/antigène est analysée dans un scanner portable. Ce scanner comprendra une matrice de LEDs ou DELs émettant à la longueur d'excitation du marquage des sondes et /ou des cibles tel qu'une LED soit disposée en dessous de chaque loge d'une plaque 96 puits ou 384 puits et plus généralement N puits. Les fonds des puits des plaques 96 puits ou N puits sont en un matériau transparent pour la longueur d'onde des LEDs. Quand différents marquages sont utilisés une LED pour chaque type d'excitation est disposée en dessous de chaque loge. Au-dessus de la plaque 96 puits ou N puits est disposé un filtre optique filtrant les longueurs d'onde d'excitation provenant des LEDs et transparent à la longueur d'onde d'émission des marquages. L'ensemble est disposé dans une chambre noire surmontée d'un capteur, caméra, CCD, appareil photo numérique, appareil argentique, avec un dispositif de mise au point sur le fond des puits et/ou la surface des plaques. Le capteur permet de réaliser une photo de la plaque à N puits. L'accumulation du signal permet de déterminer le taux de complexation de chaque cible. Dans certains modes de réalisation, le filtre est disposé en avant du capteur.

Dans certains modes de réalisation, les anticorps seront remplacés par des molécules chimériques Fab-zipper décrites dans la demande FR-A1-2 958 645. Les molécules chimériques sont marquées ou fixées sur un support préférentiellement grâce à une séquence complémentaire acide nucléique ou d'analogue d'acide nucléique fixé sur le support ou marqué par un chromophore.

Les séquences utilisées pour créer le Fab-zipper anti-Ku740 phosphorylé sur la Serine 27 sont

Chaine légère :

MMSPAQFLFLLVLWIRVSETIGDVVMTQTPLTLSVTFGQPASISCKSSQSL
LDRDGKTYLNWLLQRPGQSPKRLIYLVSKLDSGVPDRFTGSGSGTDFTLK
ISRVEAEDLGVFYCWQGTHLPQTFGGGTKLEVKRADAAPTVSIFPPSSEQ
LTSGGASVVCFLNNFYPKDINVKWKIDGSERQNGVLNSWTDQDSKDSTY
SMSSTLTLTKDEYERHNSYTCEATHKTSTDQDSKDSTYSMSSTLTLTKDE
YERHNSYTCEATHKTSTSPIVKSFNRNEC (SEQ ID3)

Chaine lourde :

MELGLSWVFLVALLNGVQCQVQLVETGGGLVRPGNSLKLSCVTSGFTFS
KYRMHWLRQFPGKRLEWIAAITVKSDNYGAHYVESVKGRFTISRDDSKSS
VYLQMNRLREEDTATYYCCTTGFAYWGQGTLVTVSAAKTTPPSVYPLAP

GSAAQTNSMVTLGCLVKGYFPEPVTVTWNSGSLSSGVHTFPAVLQSDLY
TLSSSVTVPSSTWPSETVTCNVAHPASSTKVVLQSDLYTLSSSVTVPSST
WPSETVTCNVAHPASSTKVDKKIVPRDCGCKPCICTVPEVSSVFIFPPKPK
DVLTITEEQFNSTFRSVSELPIMHQDWLNGKEFKCRVNSAAFPAPIEKTIS
KTKGRPKAPQVYTIPPPKEQMAKDKVSLTCMITDFFPEDITVEWQWNGQ
PAENYKNTQPIMDTDGSYFVYSKLNVQKSNWEAGNTFTC (SEQ ID4)

Chaine lourde alternative :

MELGLSWVFLVALLNGVQCQVQLVETGGGLVRPGNSLKLSCVTSGFTFS
KYRMHWLRQFPGKRLEWIAAITVKSDNYGAHYVESVKGRFTISRDDSKSS
VYLQMNRLREEDTATYYCCTTGFAYWGQGTLVTVSAAKTTPPSVYPLAP
GSAAQTNSMVTLGCLVKGYFPEPVTVTWNSGSLSSGVHTFPAVLQSDLY
TLSSSVTVPSSTWPSETVTCNVAHPASSTKVVLQSDLYTLSSSVTVPSST
WPSETVTCNVAHPASSTKVDKKIVPRDCGCKPC (SEQ ID5)

En C-terminal de chacune de ces séquences est fusionné un peptide : LPXTG. Le peptide LPXTG fusionné à l'une des chaines lourdes ou légère est lui-même fusionné en C-Terminal à l'extrémité 5-Terminal (N') respectivement 3-Terminale (C') d'une séquence d'acides nucléiques (ADN) ou analogues, telles que les séquences acides nucléiques soient complémentaires. En s'hybridant, elles restructurent le site actif de l'anticorps.

La figure 31 montre les résultats de l'intensité de fluorescence à partir de $\gamma$H2AX à partir de cellules irradiées à 0, 2 et 8GY (Millipore), respectivement d'un anticorps $\gamma$H2AX et d'un Fab-zipper $\gamma$H2AX.

Dans certains modes de réalisation, la molécule chimérique possède un marqueur d'adressage fait d'un acide nucléique ou d'un analogue d'acide nucléique tel que le marqueur permet l'hybridation à une position x,y précise du fond de chaque puits. Le marqueur complémentaire permet de fonctionnaliser le fond des puits. Dans certains modes de réalisation, les Fab-zipper possédant un marqueur sont complexés avec l'extrait à analyser puis l'ensemble est disposé dans un puits de la plaque de manière à ce que le marqueur puisse permettre l'adressage des Fab-zipper complexés à la cible à la position du marqueur.

Dans certains modes de réalisation, des nano ou micro particules sont munies d'un marqueur permettant la fonctionnalisation des particules par Fab-zipper comportant un marqueur complémentaire. Dans d'autres modes de réalisation, les particules comportent un second marqueur permettant de les adresser dans le fond d'une plaque 96 ou N puits. Dans d'autres modes de réalisation, le second marqueur permet de marquer la nanoparticule par un acide nucléique fluorescent particulier.

Dans certains modes de réalisation, les patchs de glue sont disposés sur le corps d'un individu (figure 32), par exemple avec un dispositif de distribution d'étiquettes, de façon à ce que les patchs soient disposés sur un ruban de distribution, et que le patch de glue soit muni d'une étiquette comportant des signes permettant d'identifier l'étiquette de manière non univoque. Par exemple, un code barre ou une succession de signes géométriques permet d'identifier le patch de manière univoque. En particulier une étiquette est composée de 4 signes géométriques, triangle, rond, carré, disposés en 5 positions successives. Par exemple, la première position indique le sens de lecture, par exemple, un triangle avec le sommet en position médiante. Les quatre positions suivantes sont une combinaison triangle haut et bas, rond, carré, ceci permettant de coder jusqu'à 256 cas. Une position supplémentaire permettant de coder 1024 cas. L'utilisation d'un caractère supplémentaire permet de coder 626 cas sur quatre positions et 3125 cas sur cinq positions.

L'utilisation d'une caméra de type kinet® et d'une analyse d'image permet de reconnaître la localisation corporelle des patchs et d'identifier son positionnement sur la plaque 96 ou N puits. Un patch peut avoir une largeur légèrement supérieure à celle d'un puits et une longueur légèrement supérieure à celle de trois puits consécutifs, de façon à ce que les poils collés à ce patch se trouvent sensiblement répartis dans trois puits consécutifs.

Exemple dans lequel des nanodiamants hydrogénés (H-NDs) sont utilisés comme support de fixation de protéines. Les nanodiamants ont un diamètre compris entre 1 et 100nm. En variante, on pourrait utiliser des microdiamants ($1\mu$m-999$\mu$m) ou des diamants submicroniques (100nm-700nm). En variante, on pourrait utiliser du charbon activé hydrogéné.

Les H-NDs ont été utilisés comme objets de capture d'un mélange de protéines provenant de deux méthodes d'extraction : une conventionnelle (tampon de lyse) et une autre basée sur les micro-ondes (15 sec à 450 W). Les cellules humaines ZR75.1, établies au laboratoire, ont été utilisées comme modèle cellulaire car elles présentent une forte induction de phosho-Ku70 après irradiation. Les cellules ont été irradiées à partir d'une source de $Cs^{137}$ (IBM637, CisBio International, Gif sur Yvette, France) à une dose de 10 Gy. Une heure après irradiation, les cellules irradiées et non irradiées ont été récupérées et comptées de manière à obtenir 1 million de cellules par condition (par tube). L'extraction est alors réalisée à la fois sur les cellules irradiées et non-irradiées. Puis 100 $\mu$g de H-NDs ont été ajoutés à l'extrait protéique et le tout a été incubé 10 mn à température ambiante et sous agitation permanente de manière à capturer les protéines à la surface des H-NDs. Puis les tubes ont été centrifugés 5 minutes à 1500 tours par minute afin de récupérer les H-NDs. Un rinçage PBS a par la suite été réalisé (centrifugation 5 minutes à 1500 tours par minute), puis les culots ont été mis en contact d'un mélange d'anticorps primaire anti-phospho-Ku70 (1/1000) et d'anticorps secondaire Alexa Fluor 488 (1/500) pendant 15 mn à 37 °c et sous agitation permanente. Les tubes ont ensuite été centrifugés 5 minutes à 1500 tours par minute afin de récupérer les H-NDs et d'éliminer l'excès d'anticorps primaire. Deux rinçages PBS ont par la suite été réalisés (centrifugation 5 minutes à 1500 tours par minute), puis les culots ont été repris dans 250 $\mu$L de PBS pour passer les échantillons sur un FACSCalibur ™ en utilisant le logiciel Cell Quest Pro au cours des acquisitions. La fluorescence de l'Alexa Fluor 488 a quant à elle été détectée sur le canal FL1 du cytomètre. Les précipitations par centrifugation peuvent être remplacées par des précipitations magnétiques en 30 secondes avec des H-NDs magnétiques comportant des cristaux de fer.

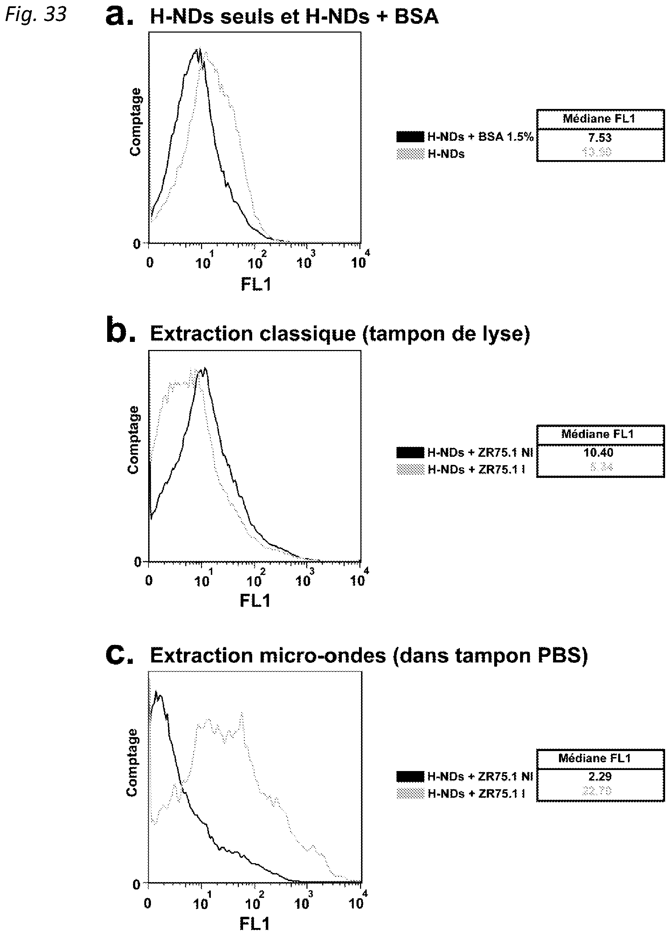

Les figures 33a, b et c illustrent les résultats obtenus de l'évolution du signal de fluorescence (FL) d'un marquage anti Ku70 en FACS pour des extraits de protéines de cellules humaines (ZR75.1) irradiées à 10Gy (gris) ou non (noir), et capturées sur particules de nanodiamants hydrogénés, avec différents mode d'extraction.

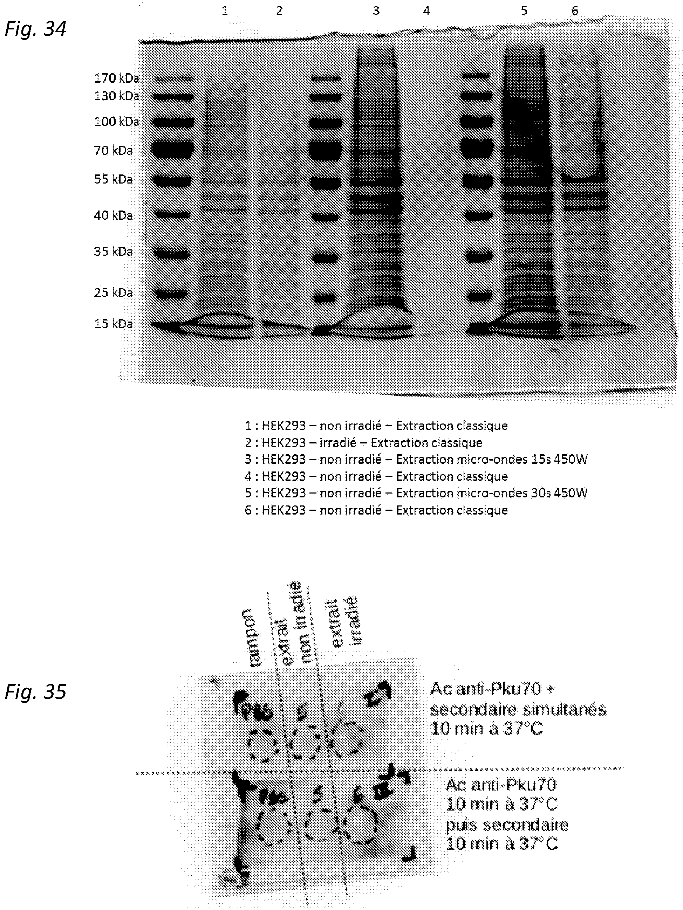

La figure 35 illustre la mise en oeuvre d'un transfert rapide du type *dot blot* pour des échantillons irradiés à 0,5 Gy, sur des membranes. Les dépôts sont réalisés sur des membranes de nitrocellulose ou de PVDF et séchés (maximum 2 min à 37°).

Les membranes sont plongées dans un bain de saturation (TBS 1X - BSA 3%) sous agitation pendant 5 min.

Les membranes sont ensuite plongées dans un bain contenant un mélange des anticorps primaires et secondaires aux bonnes dilutions (selon les fiches techniques des anticorps) dans de la solution de saturation sous agitation à 37°C pendant 10 min. Il apparaît que le mélange des anticorps primaire et secondaire donne un résultat en 10 min similaire à l'utilisation séquentielle des deux anticorps sur une durée de 20 min.

Enfin les membranes sont rincées au TBS 1X et révélées selon le type de marquage de l'anticorps secondaire (ECL ou fluorescence).

Exemple de méthode de quantification.

La méthode de quantification consiste à réaliser au moins deux mesures de la phosphorylation de protéines de la cascade ATM. Les au moins deux mesures sont réalisées par exemple sur la même protéine à au moins deux temps différents, ou sur deux protéines à un même temps, ou comprend toute combinaison de mesures à des temps différents sur des protéines différentes.

Les mesures sont reportées sur des courbes ou surfaces de référence étalon formant des gammes de dose réponse.

A la suite d'une irradiation, les cinétiques de phosphorylation/déphosphorylation de deux protéines ATM, sont telles que le taux de phosphorylation, en fonction du temps (t), par exemple H2AX(t) =$I_h$, et Ku70(t)= $I_k$ croissent dans un premier temps à des rythmes qui peuvent être différents pour les deux protéines, et dans un deuxième temps, décroissent à des rythmes qui peuvent être différents pour les deux protéines. Les cinétiques passent à leur maximum à des temps $T_{H2AX}$ et $T_{Ku70}$ qui peuvent être différents.

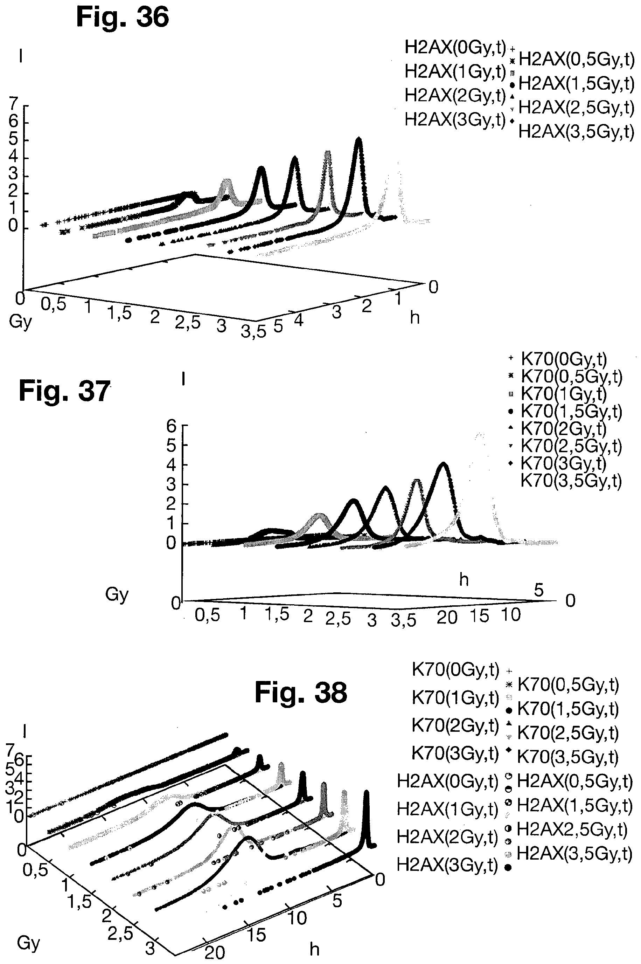

Par exemple, H2AX(t) passe à son maximum typiquement en moyenne une heure après l'irradiation alors que Ku70 passe à son maximum typiquement en moyenne 12h après l'irradiation (cf. figures 36-38).

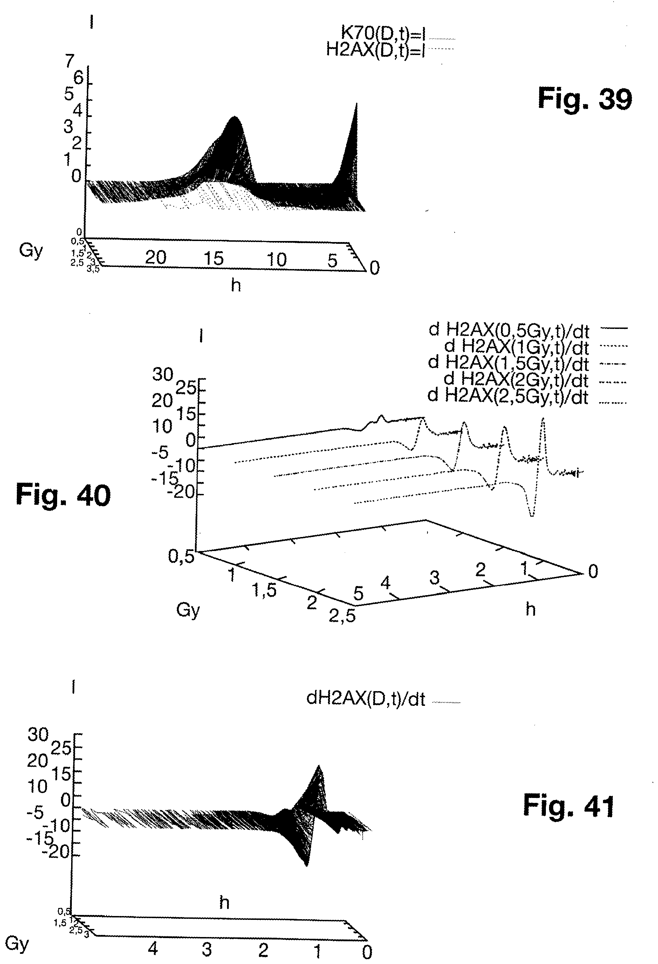

Ainsi, l'observation de la cinétique de phosphorylation/déphosphorylation en fonction de la dose (D) d'irradiation, de deux protéines ATM, telles que H2AX et Ku70, montre une dépendance définie comme H2AX(D) = $I_h$ et Ku70(D) = $I_k$ définissant ainsi une surface dans l'espace tridimensionnel (D,t,I) où à chaque coordonnées D et t correspond une coordonnée I pour chacune des protéines, telle que : H2AX(D,t) = $I_h$ et Ku70(D,t)= $I_k$ (cf. figure 39).

Ces surfaces représentent des références étalons ou gammes permettant de déterminer le taux d'irradiation d'un individu à partir d'une mesure. Elles permettent, à partir de la mesure ponctuelle i effectuée à un même moment pour deux protéines ATM présentant des cinétiques de phosphorylation différentes, de résoudre le système d'équation :

$$H2AX(D,t)=i_h$$

$$Ku70(D,t)=i_k$$

et de déterminer la dose D reçue et le moment $t_0$ de l'irradiation.

Dans certains cas, l'utilisation d'une ou deux protéines ne permet pas de converger vers une solution unique Dose/temps. Un nombre plus grand de protéines du système ATM peut alors être examiné.

Néanmoins, dans un autre mode de réalisation, les courbes de

$$H2AX(t)=I_h$$

$$H2AX(D)= I_h,$$

$$Ku70(t)=I_k,$$

$$Ku70(D)=I_k,$$

et les surfaces $H2AX(D,t)= I_h$ et $Ku70(D,t)= I_k$ peuvent être dérivées en fonction du temps et/ou de la dose tel que d $H2AX(t)/dt = I'_h$, d $Ku70(t)/dt = I'_k$,. De même, la surface peut être dérivée en fonction du temps tel que d $H2AX(D,t)/dt = I'_h$, d $Ku70(D,t)/dt = I'_k$ (cf. figures 40-41).

Par exemple, en mesurant $Ku70(D,t) = i_{k(t)}$ à un temps donné (t) et $Ku70(D,(t+\Delta t)) = i_{k(t+\Delta t)}$ avec un retard t+Δt, il est possible d'évaluer ponctuellement d $Ku70(D,t)/dt = i'_k$ par $[Ku70(D,(t+\Delta t)) - K70(D,ti)]/ \Delta t = (i_{k(t+\Delta t)} - i_{k(t)})/\Delta t$. De même, en mesurant $H2AX(D,t) = i_{h(t)}$ à un temps donné (t) et $H2AX(D,(t+\Delta t))=i_{h(t+\Delta t)}$ avec un retard t+Δt, il est possible d'évaluer ponctuellement d $H2AX(D,t)/dt = i'_h$ par $[H2AX(D,(t+\Delta t)) - H2AX(D,ti)]/ \Delta t= (i_{h(t+\Delta t)} - i_{h(t)})/\Delta t$.

Ainsi, en disposant de la surface

$$Ku70(D,t)= I_k,$$

$$H2AX(D,t)= I_h,$$

et des surfaces dérivées

$$d\ Ku70\ (D,t)/dt = I'_k,$$

$$d\ H2AX(D,t)/dt = I'_h,$$

il est possible de résoudre le système d'équation

$$d\ Ku70(D,t)/dt = (i_{k(t+\Delta t)} - i_{k(t)})/\Delta t,$$

$$Ku70(D,t) = i_k,$$

$$d\,H2AX(D,t)/dt = (i_{h(t+\Delta t)} - i_{h(t)})/\Delta t,$$

$$H2AX(D,t) = i_h,$$

et de lever les éventuelles incertitudes avec une solution unique déterminant la dose reçue et le moment de l'irradiation à partir de l'observation du taux de phosphorylation d'une protéine et la variation de ce taux dans un court laps de temps.

La précision de l'observation sera améliorée en observant à la fois plusieurs protéines telles que de préférence des protéines ayant une réponse rapide comme H2AX avec un maximum de réponse par exemple autour d'une 1 heure et Ku70 avec un maximum de réponse par exemple autour de 12 h

## Revendications

1. Procédé d'évaluation par biodosimétrie de la dose d'irradiation reçue par un individu ayant été soumis à un rayonnement ionisant, comprenant les étapes consistant à :

   a) prélever, dans au moins une zone du corps de l'individu, des bulbes ou des follicules pileux de poils (1) et/ou de cheveux de l'individu,
   b) extraire des protéines (51) de cellules de ces bulbes ou follicules pileux prélevés, ces protéines comprenant des protéines de la cascade ATM ayant subies une phosphorylation et/ou un acétylation induite par le rayonnement ionisant,
   c) analyser au moins deux types de protéines extraites, et interpréter les résultats d'analyse afin de déterminer la dose d'irradiation reçue dans la ou chaque zone de prélèvement, les protéines phosphorylées et/ou acétylées de la cascade ATM étant destinées à produire des signaux lors de l'analyse, dont une grandeur telle qu'une intensité ou un ratio d'intensités permet d'évaluer cette dose d'irradiation,

   ledit procédé étant **caractérisé en ce que** lesdits au moins deux types de protéines présentent des cinétiques de phosphorylation/déphosphorylation différentes,
   et **en ce que** l'étape c) d'analyse et d'interprétation comprend les sous-étapes consistant à :

   - soumettre chaque type de protéines à un marquage, la forme phosphorylée de chaque type de protéines pouvant éventuellement avoir un marquage spécifique,
   - soumettre les protéines à une analyse dans laquelle au moins un signal induit par le marquage du type de protéines est étudié,

- comparer une grandeur ou un paramètre, telle que l'intensité, de ce signal à une courbe d'étalonnage représentant l'évolution de cette grandeur en fonction d'une dose d'irradiation reçue par un individu, et
- en déduire la dose d'irradiation reçue par l'individu, dans la zone de prélèvement.

**2.** Procédé selon la revendication 1 , **caractérisé en ce que** les protéines sont immobilisées sur un support à l'étape c), les protéines étant fixées directement ou indirectement audit support.

**3.** Procédé selon la revendication 1 ou 2, **caractérisé en ce qu'**au moins les protéines H2AX et Ku70 sont analysées à l'étape c).

**4.** Procédé selon l'une des revendications précédentes, **caractérisé en ce que** des follicules pileux ou bulbes de poils (1) et/ou de cheveux sont prélevés dans plusieurs zones distinctes du corps, de façon à déterminer la dose d'irradiation reçue par chaque zone.

**5.** Procédé selon l'une des revendications précédentes, **caractérisé en ce que** l'étape a) de prélèvement comprend les sous-étapes consistant à :

- appliquer un patch (10, 20) sur la ou chaque zone, et le retirer pour arracher des poils (1) et/ou des cheveux avec leurs follicules pileux ou bulbes dans cette zone, et
- introduire le patch et les poils et/ou cheveux avec leurs follicules pileux ou bulbes dans un puits de réaction (3).

**6.** Procédé selon l'une des revendications précédentes, **caractérisé en ce qu'**il comprend, en outre, les étapes consistant à :

- prélever du sang de l'individu, par exemple à l'extrémité d'un doigt,
- récupérer les cellules du sang prélevé, à l'exception des globules rouges, et extraire des protéines de ces cellules, ces protéines comprenant des protéines du système ATM ayant subies une phosphorylation et/ou un acétylation induite par le rayonnement ionisant.

**7.** Procédé selon l'une des revendications précédentes, **caractérisé en ce que** l'extraction des protéines comprend les sous-étapes consistant à :

- mettre en contact ou mélanger les follicules pileux prélevés et/ou le liquide résiduel récupéré avec une solution de lyse cellulaire, ou
- mettre en contact ou mélanger les cellules avec une solution non dénaturante, de préférence exempte de phosphore, puis soumettre l'ensemble à une énergie mécanique, par micro-onde ou par ultrason, destinée à provoquer la lyse des cellules.

**8.** Procédé selon l'une des revendications précédentes, **caractérisé en ce que** différents prélèvements sont répartis dans différents puits de réaction (32) d'une plaque multi-puits (30), et **en ce que** le fond (34, 50) des puits (32) est formé par une membrane (35), éventuellement filtrante, sur laquelle les protéines (51) sont destinées à se déposer ou à se fixer, directement ou par l'intermédiaire d'anticorps (52) spécifiques préalablement fixés sur le fond ou à des particules, ce fond étant de préférence amovible.

**9.** Procédé selon l'une des revendications précédentes, **caractérisé en ce qu'**il est appliqué à plusieurs individus, dans un lieu habituellement non équipé d'appareils de laboratoire.

**10.** Procédé selon la revendication précédente, **caractérisé en ce qu'**il comprend une étape préliminaire d'identification du ou de chaque individu.

**11.** Procédé selon l'une des revendications précédentes, dans lequel la dose d'irradiation ou le moment de l'irradiation est déterminée au moyen d'une surface répertoriant la variation du taux de phosphorylation d'au moins une protéine en fonction de la dose d'irradiation reçue et du temps de retard de l'observation.

**12.** Procédé selon la revendication précédente, dans lequel la dose d'irradiation ou le moment de l'irradiation est déterminée au moyen d'une dérivée ou d'un accroissement fini de ladite surface.

**Patentansprüche**

1.  Verfahren zur Beurteilung, mittels Biodosimetrie, der von einer Person unter dem Einfluss von ionisierender Strahlung aufgenommenen Strahlendosis umfassend die Schritte, die bestehen im:

    a) Entnehmen, an mindestens einer Zone des Körpers der Person, der Zwiebeln oder der Haarfollikel von Körperhaaren (1) und/oder von Kopfhaaren der Person,
    b) Extrahieren der Proteine (51) aus Zellen dieser entnommenen Zwiebeln oder Haarfollikel, wobei diese Proteine Proteine der ATM-Kaskade umfassen, die eine Phosphorylierung und/oder eine durch die ionisierende Strahlung induzierte Acetylierung erlitten haben,
    c) Analysieren von mindestens zwei Arten extrahierter Proteine, und Auswerten der Analyseergebnisse, um die an der oder jeder Entnahmezone aufgenommene Strahlendosis zu bestimmen, wobei die phosphorylierten und/oder acetylierten Proteine der ATM-Kaskade dazu vorgesehen sind, bei der Analyse Signale zu erzeugen, von denen eine Größe wie etwa eine Intensität oder ein Intensitätsverhältnis es ermöglicht, diese Strahlendosis zu beurteilen,

    wobei das Verfahren **dadurch gekennzeichnet ist, dass** die mindestens zwei Proteinarten verschiedene Phosphorylierungs-/Dephosphorylierungskinetiken aufweisen,
    und dadurch, dass der Schritt c) des Analysierens und des Auswertens die Teilschritte umfasst, die bestehen im:

    - Aussetzen jeder Proteinart einer Markierung, wobei die phosphorylierte Form jeder Proteinart gegebenenfalls eine spezifische Markierung aufweisen kann,
    - Aussetzen der Proteine einer Analyse, wobei mindestens ein durch die Markierung des Proteintyps induziertes Signal untersucht wird,
    - Vergleichen einer Größe oder eines Parameters, wie etwa der Intensität dieses Signals mit einer Eichkurve, die die Entwicklung dieser Größe in Abhängigkeit von einer von einer Person aufgenommenen Strahlendosis wiedergibt, und
    - Ableiten daraus der Strahlendosis, die von der Person an der Entnahmezone aufgenommen wurde.

2.  Verfahren nach Anspruch 1, **dadurch gekennzeichnet, dass** die Proteine im Schritt c) auf einem Träger immobilisiert werden, wobei die Proteine direkt oder indirekt am Träger fixiert werden.

3.  Verfahren nach Anspruch 1 oder 2, **dadurch gekennzeichnet, dass** im Schritt c) mindestens die Proteine H2AX und Ku70 analysiert werden.

4.  Verfahren nach einem der vorstehenden Ansprüche, **dadurch gekennzeichnet, dass** Haarfollikel oder Zwiebeln von Körperhaaren (1) und/oder von Kopfhaaren an mehreren unterschiedlichen Zonen des Körpers entnommen werden, um die von jeder Zone aufgenommene Strahlendosis zu bestimmen.

5.  Verfahren nach einem der vorstehenden Ansprüche, **dadurch gekennzeichnet, dass** der Schritt a) des Entnehmens die Teilschritte umfasst, die bestehen im:

    - Aufbringen eines Pflasters (10, 20) auf die oder jede Zone, und Abziehen desselben, um Körperhaare (1) und/oder Kopfhaare mit ihren Haarfollikeln oder Zwiebeln an dieser Zone auszureißen, und
    - Einbringen des Pflasters und der Körperhaare und/oder Kopfhaare mit ihren Haarfollikeln oder Zwiebeln in eine Reaktionsvertiefung (3).

6.  Verfahren nach einem der vorstehenden Ansprüche, **dadurch gekennzeichnet, dass** es weiter die Schritte umfasst, die bestehen im:

    - Entnehmen von Blut der Person, zum Beispiel am Ende eines Fingers,
    - Gewinnen der Zellen des entnommenen Blutes, mit Ausnahme der roten Blutkörperchen, und Extrahieren der Proteine aus diesen Zellen, wobei diese Proteine des ATM-Systems umfassen, die eine induzierte Phosphorylierung und/oder eine durch die ionisierende Strahlung Acetylierung erlitten haben.

7.  Verfahren nach einem der vorstehenden Ansprüche, **dadurch gekennzeichnet, dass** das Extrahieren der Proteine die Teilschritte umfasst, die bestehen im:

- Inkontaktbringen oder Mischen der entnommenen Haarfollikel und/oder der gewonnenen Restflüssigkeit mit einer Zelllyselösung, oder
- Inkontaktbringen oder Mischen der Zellen mit einer nicht denaturierenden Lösung, die vorzugsweise frei von Phosphor ist, und anschließend Aussetzen der Gesamtheit einer mechanischen Energie durch Mikrowelle oder durch Ultraschall, die dazu vorgesehen ist, die Lyse der Zellen zu provozieren.

8. Verfahren nach einem der vorstehenden Ansprüche, **dadurch gekennzeichnet, dass** verschiedene Entnahmen in verschiedene Reaktionsvertiefungen (32) einer Platte mit mehreren Vertiefungen (30) verteilt werden, und dadurch, dass der Boden (34, 50) der Vertiefungen (32) von einer gegebenenfalls filternden Membran (35) gebildet wird, auf der die Proteine (51) vorgesehen sind, sich abzusetzen oder sich direkt oder mithilfe von spezifischen Antikörpern (52), die zuvor am Boden oder an Teilchen fixiert wurden, zu fixieren, wobei dieser Boden vorzugsweise abnehmbar ist.

9. Verfahren nach einem der vorstehenden Ansprüche, **dadurch gekennzeichnet, dass** es auf mehrere Personen an einem Ort angewandt wird, der üblicherweise nicht mit Laborgeräten ausgestattet ist.

10. Verfahren nach dem vorstehenden Anspruch, **dadurch gekennzeichnet, dass** es einen vorausgehenden Schritt des Identifizierens der oder jeder Person umfasst.

11. Verfahren nach einem der vorstehenden Ansprüche, wobei die Strahlendosis oder der Moment der Bestrahlung mittels einer Oberfläche bestimmt wird, die die Variation des Phosphorylierungsgrades von mindestens einem Protein in Abhängigkeit von der aufgenommenen Strahlendosis und der Verzögerungszeit der Beobachtung repertorisiert.

12. Verfahren nach dem vorstehenden Anspruch, wobei die Strahlendosis oder der Moment der Bestrahlung mittels einer Ableitung oder eines endlichen Zuwachses der Oberfläche bestimmt wird.

**Claims**

1. A method for the evaluation by biodosimetry of the irradiation dose received by an individual who has been subjected to ionising radiation, comprising the steps consisting of:

   a) sampling, in at least one region of the body of the individual, hair bulbs or follicles of body and/or head hair (1) of the individual,
   b) extracting proteins (51) from cells of these hair bulbs or follicles sampled, these proteins comprising proteins of the ATM cascade that have undergone phosphorylation and/or acetylation caused by the ionising radiation,
   c) analysing at least two types of protein extracted, and interpreting the analysis results in order to determine the irradiation dose received in the or each sampling region, the phosphorylated and/or acetylated proteins of the ATM cascade being intended to produce signals during the analysis, a quantity of which such as an intensity ratio allowing to evaluate this irradiation dose,

   said method being **characterised in that** said at least two types of protein have different phosphorylation/dephosphorylation kinetics,
   and **in that** the analysis and interpretation step c) comprises substeps consisting of:

   - subjecting each type of protein to marking, the phosphorylated form of each type of protein may optionally have a specific marking,
   - subjecting the proteins to an analysis wherein at least one signal caused by the marking of the protein type is studied,
   - comparing a quantity or a parameter, such as the intensity, of this signal with a calibration curve representing the change in this quantity as a function of an irradiation dose received by an individual, and
   - deducing therefrom the irradiation dose received by the individual, in the sampling region.

2. The method according to claim 1, **characterised in that** the proteins are immobilised on a support at step c), the proteins being attached directly or indirectly to said support.

3. The method according to claim 1 or claim 2, **characterised in that** at least the H2AX and Ku70 proteins are analysed at step c).

4. The method according to one of the preceding claims, **characterised in that** the hair follicles or bulbs of body and/or head hair (1) are sampled from a plurality of separate regions of the body, so as to determine the irradiation dose received by each region.

5. The method according to one of the preceding claims, **characterised in that** the sampling step a) comprises the substeps consisting of:

- applying a patch (10, 20) to the or each region, and removing it in order to pull off the body and/or head hairs (1) with their hair follicles or bulbs in this region, and
- introducing the patch and the body and/or head hair with the hair follicles or bulbs into a reaction well (3).

6. The method according to one of the preceding claims, **characterised in that** it further comprises the steps consisting of:

- sampling the blood of the individual, for example at the end of a finger,
- recovering the blood cells sampled, with the exception of the red corpuscles, and extracting proteins from these cells, these proteins comprising proteins of the ATM system that have undergone phosphorylation and/or acetylation caused by the ionising radiation.

7. The method according to one of the preceding claims, **characterised in that** the extraction of the proteins comprises substeps consisting of:

- contacting or mixing the hair follicles sampled and/or the residual liquid recovered with a cell lysis solution, or
- contacting or mixing the cells with a non-denaturising solution, preferably free from phosphorus, and then subjecting the whole to a mechanical energy, by microwave or ultrasound, intended to cause lysis of the cells.

8. The method according to one of the preceding claims, **characterised in that** various samples are distributed in various reaction wells (32) of a multiwell plate (30), and **in that** the bottom (34, 50) of the wells (32) is formed by a membrane (35), optionally filtering, on which the proteins (51) are intended to be deposited or attached, directly or by means of specific antibodies (52) previously attached to the bottom or to particles, this bottom preferably being removable.

9. The method according to one of the preceding claims, **characterised in that** it is applied to a plurality of individuals, in a place usually not equipped with laboratory apparatus.

10. The method according to the preceding claim, **characterised in that** it comprises a preliminary step of identifying the or each individual.

11. The method according to one of the preceding claims, wherein the irradiation dose or the moment of irradiation is determined by means of a surface listing the variation in the degree of phosphorylation of at least one protein as a function of the irradiation dose received and the delay time of the observation.

12. The method according to the preceding claim, wherein the irradiation dose or the moment of irradiation is determined by means of a derivative or a finite increase of said surface.

Fig. 1

Fig. 2

Fig. 3

Fig. 4

Fig. 5

Fig. 6

Fig. 8

Fig. 7

Fig. 9

*Fig. 10*

*Fig. 11*

*Fig. 12*

*Fig. 13*

*1ère plaque*

*2ème plaque*

**Fig. 14**

**Fig. 15**

**Fig. 16**

Fig. 17

Fig. 18

*Fig. 19*

*Fig. 20*

*Fig. 21*

Intensité du signal

Silicium

Phosphore

Magnésium

Longueur d'onde (nm)

*Fig. 22*

Puce γ - H2AX

Moyenne sommes intensité (U.A.)

$R^2 = 0,96$

2500000
2000000
1500000
1000000
500000
0

0,00E+00   1,00E+00   2,00E+00   3,00E+00   4,00E+00   5,00E+00

Dose d'irradiation (Gy)

*Fig. 23*

0        2        6        8     Gy

*Fig. 24*

Intensité de fluorescence (U.A.)

16000

12000

8000

4000

0

0        2        4        6        8

Dose d'irradiation (Gy)

*Fig. 25*

Fig.26

Neocarzinostatine (NCS)

Fig. 27

ν -rays

Fig. 28

*Fig. 29*

A)

B)

*Fig. 31*

*Fig. 30*

*Fig. 32*

*Fig. 33* **a.** H-NDs seuls et H-NDs + BSA

| Médiane FL1 |
| --- |
| H-NDs + BSA 1.5% | 7.53 |
| H-NDs | |

**b.** Extraction classique (tampon de lyse)

| Médiane FL1 |
| --- |
| H-NDs + ZR75.1 NI | 10.40 |
| H-NDs + ZR75.1 I | |

**c.** Extraction micro-ondes (dans tampon PBS)

| Médiane FL1 |
| --- |
| H-NDs + ZR75.1 NI | 2.29 |
| H-NDs + ZR75.1 I | |

*Fig. 34*

1 : HEK293 – non irradié – Extraction classique
2 : HEK293 – irradié – Extraction classique
3 : HEK293 – non irradié – Extraction micro-ondes 15s 450W
4 : HEK293 – non irradié – Extraction classique
5 : HEK293 – non irradié – Extraction micro-ondes 30s 450W
6 : HEK293 – non irradié – Extraction classique

*Fig. 35*

## Fig. 36

H2AX(0Gy,t) +
H2AX(1Gy,t) *
H2AX(2Gy,t) •
H2AX(3Gy,t) ◆

H2AX(0,5Gy,t)
H2AX(1,5Gy,t)
H2AX(2,5Gy,t)
H2AX(3,5Gy,t)

## Fig. 37

+ K70(0Gy,t)
* K70(0,5Gy,t)
▓ K70(1Gy,t)
• K70(1,5Gy,t)
▲ K70(2Gy,t)
▾ K70(2,5Gy,t)
◆ K70(3Gy,t)
K70(3,5Gy,t)

## Fig. 38

K70(0Gy,t)
K70(1Gy,t)
K70(2Gy,t)
K70(3Gy,t)
H2AX(0Gy,t)
H2AX(1Gy,t)
H2AX(2Gy,t)
H2AX(3Gy,t)

K70(0,5Gy,t)
K70(1,5Gy,t)
K70(2,5Gy,t)
K70(3,5Gy,t)
H2AX(0,5Gy,t)
H2AX(1,5Gy,t)
H2AX2,5Gy,t)
H2AX(3,5Gy,t)

I

K70(D,t)=I
H2AX(D,t)=I

**Fig. 39**

7
6
5
4
3
2
1
0

Gy

0
0,5
1,5
2,5 3,5

20    15    10    5    0

h

I

d H2AX(0,5Gy,t)/dt ⎯⎯
d H2AX(1Gy,t)/dt ⋯⋯
d H2AX(1,5Gy,t)/dt ⎯·⎯
d H2AX(2Gy,t)/dt ⎯⎯⎯
d H2AX(2,5Gy,t)/dt ⋯⋯

**Fig. 40**

30
20
25
10
15
0
-5
-10
-15
-20

0,5

1

1,5

2

2,5

5    4    3    2    1    0

Gy

h

I

dH2AX(D,t)/dt ⎯⎯

**Fig. 41**

30
20
25
10
15
0
-5
-10
-15
-20

h

0,5
1,5 1
2,5 3

Gy

4    3    2    1    0

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2011050812 W

- FR 2958645 A1

**Littérature non-brevet citée dans la description**

- **ROGAKOU et al.** *J. biol. Chem.,* 1998, vol. 273, 5858-5868
- **PAULL et al.** *Curr. Biol.,* 2000, vol. 10, 886-895
- **DE CHRISTOPHE E. REDON et al.** The Use of Gamma-H2AX as a Biodosimeter for Total-Body Radiation Exposure in Non-Human Primates. *PLOS ONE,* 23 Novembre 2010, vol. 5, e15544

- Qγ-H2AX, an analysis method for partial-body radiation exposure using γ-H2AX in non-human primate lymphocytes. **DE CHRISTOPHE E. REDON et al.** Radiation Measurements. ELSEVIER, 25 Février 2011, vol. 46, 877-881

Sequence listing.txt

Listage des séquences

- - - - - - - - - - - - - - - - - - - - - - - - - - - - -

<110> Commissariat à l'énergie atomique et aux énergies alternatives

<120> Procédé d'évaluation par biodosimétrie de la dose d'irradiation reçue par un individu ayant été soumis à un rayonnement ionisant

<130> PAT2113540FR00

<140> FR1354347

<141> 15-05-2013

<160> 5

<170> PatentIn version 3.5.1.

<210> 1

<211> 10

<212> ADN

<213> séquence artificielle

<220>

<223> épitope phosphorylé, la sérine porte un groupement phosphate

<400> 1

ENLEASGDYK          10

<210> 2

<211> 10

<212> ADN

<213> Séquence artificielle

<220>

<223> épitope non phosphorylé

<400> 2

ENLEASGDYK          10

<210> 3

<211> 279

<212> ADN

<213> Séquence artificielle

<220>

<223> Chaîne légère. Un peptide LPXTG est fusionné à l'extrémité C-terminale de la séquence artificielle et le peptide LPXTG est lui-même fusionné en c-terminale à l'extrémité 3' d'une séquence d'acides nucléiques (ADN) ou analogue.

<400> 3

```
MMSPAQFLFL  LVLWIRVSET  IGDVVMTQTP  LTLSVTFGQP  ASISCKSSQS  LLDRDGKTYL      60
NWLLQRPGQS  PKRLIYLVSK  LDSGVPDRFT  GSGSGTDFTL  KISRVEAEDL  GVFYCWQGTH     120
LPQTFGGGTK  LEVKRADAAP  TVSIFPPSSE  QLTSGGASVV  CFLNNFYPKD  INVKWKIDGS     180
ERQNGVLNSW  TDQDSKDSTY  SMSSTLTLTK  DEYERHNSYT  CEATHKTSTD  QDSKDSTYSM     240
SSTLTLTKDE  YERHNSYTCE  ATHKTSTSPI  VKSFNRNEC          279
```

<210> 4
<211> 437
<212> ADN
<213> séquence artificielle
<220>
<223> Chaîne lourde. Un peptide LPXTG est fusionné à l'extrémité C-terminale de la séquence artificielle et le peptide LPXTG est lui-même fusionné en c-terminale à l'extrémité 5' d'une séquence d'acides nucléiques (ADN) ou analogue.
<400> 4

```
MELGLSWVFL VALLNGVQCQ VQLVETGGGL VRPGNSLKLS CVTSGFTFSK YRMHWLRQFP          60
GKRLEWIAAI TVKSDNYGAH YVESVKGRFT ISRDDSKSSV YLQMNRLREE DTATYYCCTT         120
GFAYWGQGTL VTVSAAKTTP PSVYPLAPGS AAQTNSMVTL GCLVKGYFPE PVTVTWNSGS         180
LSSGVHTFPA VLQSDLYTLS SSVTVPSSTW PSETVTCNVA HPASSTKVVL QSDLYTLSSS         240
VTVPSSTWPS ETVTCNVAHP ASSTKVDKKI VPRDCGCKPC ICTVPEVSSV FIFPPKPKDV         300
LTITEEQFNS TFRSVSELPI MHQDWLNGKE FKCRVNSAAF PAPIEKTISK TKGRPKAPQV         360
YTIPPPKEQM AKDKVSLTCM ITDFFPEDIT VEWQWNGQPA ENYKNTQPIM DTDGSYFVYS         420
KLNVQKSNWE AGNTFTC          437
```

<210> 5
<211> 280
<212> ADN
<213> Séquence artificielle
<220>
<223> Chaîne lourde alternative. Un peptide LPXTG est fusionné à l'extrémité C-terminale de la séquence artificielle et le peptide LPXTG est lui-même fusionné en C-terminale à l'extrémité 5' d'une séquence d'acides nucléiques (ADN) ou analogue.
<400> 5

```
MELGLSWVFL VALLNGVQCQ VQLVETGGGL VRPGNSLKLS CVTSGFTFSK YRMHWLRQFP          60
GKRLEWIAAI TVKSDNYGAH YVESVKGRFT ISRDDSKSSV YLQMNRLREE DTATYYCCTT         120
GFAYWGQGTL VTVSAAKTTP PSVYPLAPGS AAQTNSMVTL GCLVKGYFPE PVTVTWNSGS         180
LSSGVHTFPA VLQSDLYTLS SSVTVPSSTW PSETVTCNVA HPASSTKVVL QSDLYTLSSS         240
VTVPSSTWPS ETVTCNVAHP ASSTKVDKKI VPRDCGCKPC          280
```